(19) Europäisches Patentamt
European Patent Office
Office européen des brevets

(11) Publication number: 0 034 945
B1

(12) **EUROPEAN PATENT SPECIFICATION**

(45) Date of publication of patent specification: **07.08.85**

(21) Application number: **81300767.1**

(22) Date of filing: **24.02.81**

(51) Int. Cl.[4]: **C 07 D 231/38,** A 01 N 43/56
// C07C109/00, C07C87/52,
C07C87/60, C07C121/78,
C07C79/12, C07C121/52

(54) **N-phenylpyrazole derivatives.**

(30) Priority: **26.02.80 GB 8006428**

(43) Date of publication of application:
**02.09.81 Bulletin 81/35**

(45) Publication of the grant of the patent:
**07.08.85 Bulletin 85/32**

(84) Designated Contracting States:
**AT BE CH DE FR GB IT LI LU NL SE**

(56) References cited:
**EP-A-0 002 180**
**EP-A-0 026 034**
**CH-A- 365 730**
**US-A-3 336 285**
**US-A-3 760 082**
**US-A-4 066 776**
**US-A-4 145 554**

**Eur. J. MED. CHEM (1979), 14(6) pp. 539-541**

(73) Proprietor: **MAY & BAKER LIMITED**
**Dagenham Essex, RM10 7 XS (GB)**

(72) Inventor: **Hatton, Leslie Roy**
**558 Galleywood Road**
**Chelmsford Essex (GB)**
Inventor: **Parnell, Edgar William**
**10 Brookside Emerson Park**
**Hornchurch Essex (GB)**
Inventor: **Roberts, David Alan**
**78 Brickhill Drive**
**Bedford Bedfordshire (GB)**

(74) Representative: **Kelly, Hubert Francis et al**
**J.A. Kemp & Co. 14 South Square Gray's Inn**
**London WC1R 5EU (GB)**

**EP 0 034 945 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to N-phenylpyrazole derivatives, compositions containing them and their use as herbicides.

In J. Heter. Chem., *12* (1975), 1199—1205, P. L. Southwick and B. Dhawan have described experiments for the preparation of 4,6-diaminopyrazolo[3,4-*d*]-pyrimidines in the expectation that such pyrimidine derivatives would have useful pharmacological properties. They employed as starting materials 1-phenyl-5-amino-4-cyanopyrazoles of the general formula:

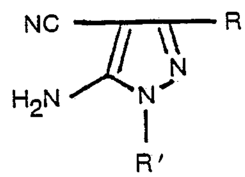

I

wherein R represents *inter alia* a hydrogen atom, and R′ represents a hydrogen atom, a methyl group, a hydroxyethyl group or a phenyl group substituted by one or more chlorine atoms and/or methyl groups. Included amongst numerous pyrazole compounds prepared and disclosed by Southwick and Dhawan were 5-amino-4-cyano-1-(2,4-dichlorophenyl)pyrazole and 5-amino-4-cyano-1-(4-chloro-2-methylphenyl)-pyrazole. This publication contains no suggestion that compounds of general formula I possess or would be expected to possess herbicidal activity.

Apparently these pyrazole compounds did not lead (according to the authors of the article) to useful therapeutic (viz. antimalarial) 4,6-diamino-pyrazolo[3,4-*d*]pyrimidines.

In Eur. J. Med. Chem. *14* (1979), No. 6, 539—541, A. Kreutzberger and K. Burgwitz have described the preparation of 5-amino-4-cyano-1-(2,4,5-trichlorophenyl)pyrazole, which is stated to exhibit antibacterial inhibitory activity against *Escherichia coli.* This publication contains no suggestion that 5-amino-4-cyano-1-(2,4,5-trichlorophenyl)pyrazole possesses or would be expected to possess herbicidal activity.

Moreover, in United States Patent No. 3336285 (E. B. Towne *et al*) there is disclosed 5-amino-4-cyano-1-(2,4-dinitrophenyl)pyrazole [alternatively named in the U.S. patent as 2-(2,4-dinitrophenyl)-3-amino-4-cyanopyrazole] as a starting material for the preparation of monoazo compounds useful as dyes or colouring agents.

In Japanese Patent Application 29598/63 (applied for by Takeda Chemical Industries Ltd: Publication No. 19958/65) there are disclosed pyrazole derivatives of the general formula:

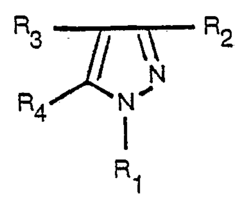

II

(wherein $R_1$ represents a hydrogen atom or an unsubstituted phenyl group, $R_2$ represents a hydrogen atom or a lower alkyl group, $R_3$ represents a hydrogen or halogen atom, or a nitro or cyano group, and $R_4$ represents a lower alkyl group, an amino group or a lower alkoxy group) which are useful as herbicides.

It has now unexpectedly been found after extensive research and experimentation that when the substituent $R_1$ on the pyrazole ring of compounds of general formula II is a phenyl radical carrying particular substituents, $R_2$ represents a hydrogen atom, $R_3$ represents a cyano or substituted carbamoyl radical, and $R_4$ represents an amino group, the compounds also have useful herbicidal activity and have unexpectedly advantageous herbicidal properties in relation to particular compounds disclosed in Japanese Patent Application 29598/63 (Publication No. 19958/65 — Derwent Basic No. G 3904), e.g. the closely related compound 1-phenyl-4-cyano-5-aminopyrazole.

The present invention accordingly provides, as herbicides, N-phenylpyrazole derivatives of the general formula:—

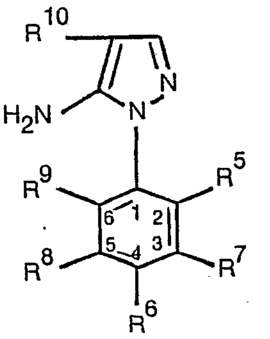

III

wherein each of the symbols $R^5$ and $R^6$, which may be the same or different, represents an alkyl or alkoxy radical containing from 1 to 4 carbon atoms, a trifluoromethyl, trifluoromethoxy, nitro, cyano or primary amino radical, or a fluorine, chlorine or bromine atom, each of the symbols $R^7$, $R^8$ and $R^9$, which may be the same or different, represents a hydrogen atom, an alkyl or alkoxy radical containing from 1 to 4 carbon atoms, a trifluoromethyl, trifluoromethoxy, nitro, cyano or primary amino radical or a fluorine, chlorine or bromine atom, or the symbols $R^5$, $R^7$, $R^8$ and $R^9$ each represents a hydrogen atom and the symbol $R^6$ represents a trifluoromethoxy or, preferably, a trifluoromethyl radical, and the symbol $R^{10}$ represents a cyano radical or substituted carbamoyl radical —$CONHR^{11}$ (wherein $R^{11}$ represents a methyl or ethyl radical) with the proviso that when $R^{10}$ represents a cyano radical $R^5$ is other than a chlorine atom when $R^6$ and $R^9$ each represent a chlorine atom and $R^7$ and $R^8$ each represent a hydrogen atom, and, when at least one of symbols $R^5$, $R^6$, $R^7$, $R^8$ and $R^9$ represents a primary amino radical, agriculturally acceptable acid addition salts thereof. Preferably each of the symbols $R^5$ and $R^6$ represents an alkyl radical containing from 1 to 4 carbon atoms, a trifluoromethyl or nitro radical or a fluorine, chlorine or bromine atom; preferably $R^7$ represents a hydrogen atom, an alkyl or alkoxy radical containing from 1 to 4 carbon atoms, a cyano radical or a fluorine, chlorine or bromine atom; preferably each of the symbols $R^8$ and $R^9$ represents a hydrogen atom or a fluorine, chlorine or bromine atom, and preferably $R^{10}$ represents a cyano radical.

The N-phenylpyrazole derivative excluded from the scope of general formula III by the proviso indicated heretofore, viz 5-amino-4-cyano-1-(2,4,6-trichlorophenyl)pyrazole, its salts, compositions containing it, its use as a herbicide and its preparation by reacting 2,4,6-trichlorophenylhydrazine with a lower alkoxymethylenemalononitrile is described and claimed in European Patent Application 026034 (filed on 4th August 1980).

It is to be understood that in the present specification and accompanying claims the alkyl and alkoxy radicals within the definitions of the symbols $R^5$, $R^6$, $R^7$, $R^8$ and $R^9$ may be straight- or branched-chained. Preferably the alkyl radicals within the definitions of the symbols $R^5$, $R^6$, $R^7$, $R^8$ and $R^9$ are methyl; preferably the symbol $R^{11}$ represents methyl, and preferably the alkoxy radicals within the definitions of the symbols $R^5$, $R^6$, $R^7$, $R^8$ and $R^9$ are methoxy.

Particularly preferred compounds of general formula III as herbicides are those wherein each of $R^5$ and $R^6$ are as hereinbefore defined and more particularly represents an alkyl radical containing from 1 to 4 carbon atoms, a trifluoromethyl or nitro radical or a fluorine, chlorine or bromine atom, and more especially a methyl, trifluoromethyl or nitro radical or a fluorine, chlorine or bromine atom, $R^7$ is as hereinbefore defined and more particularly represents a hydrogen atom or, preferably, an alkyl or alkoxy radical containing from 1 to 4 carbon atoms, a cyano radical or a fluorine, chlorine or bromine atom, and more especially a methyl, methoxy or cyano radical, or a fluorine, chlorine or bromine atom, $R^8$ and $R^9$ represent hydrogen atoms, and $R^{10}$ is as hereinbefore defined and more particularly represents a cyano radical, and more especially compounds of general formula III hereinbefore defined as being particularly preferred wherein at least one of the symbols $R^5$, $R^6$ and $R^7$ represents a chlorine atom. 5-Amino-4-cyano-1-(2,3,4-trichlorophenyl)pyrazole is of outstanding importance as a herbicide.

By the term 'agriculturally acceptable acid addition salts' as used in the present specification is meant salts the anions of which are known and accepted in the art for the formation of salts of pesticidally active bases for agricultural or horticultural use. Suitable acid addition salts of the compounds of general formula III, wherein at least one of the symbols $R^5$, $R^6$, $R^7$, $R^8$ and $R^9$ represents a primary amino radical, include salts with inorganic acids, for example hydrochlorides, sulphates, phosphates and nitrates and salts with organic acids, for example acetic acid. It is to be understood that where reference is made in the present specification to the compounds of general formula III, such reference is intended to include also the agriculturally acceptable acid addition salts of compounds of general formula III, where appropriate.

The following compounds of general formula III are of particular interest as herbicides:—

Compound

A 5-amino-4-cyano-1-(2,3,4-trichlorophenyl)pyrazole

B 5-amino-4-cyano-1-(2-nitro-4-trifluoromethylphenyl)pyrazole

| Compound | |
|---|---|
| C | 5-amino-4-cyano-1-(2,4-dichlorophenyl)pyrazole |
| D | 5-amino-1-(2-bromo-3,4-dichlorophenyl)-4-cyanopyrazole |
| E | 5-amino-4-cyano-1-(3,4-dichloro-2-methylphenyl)pyrazole |
| F | 5-amino-1-(3-bromo-2,4-dichlorophenyl)-4-cyanopyrazole |
| G | 5-amino-4-cyano-1-(2,4-dichloro-3-methylphenyl)pyrazole |
| H | 5-amino-4-cyano-1-(2,4-dichloro-3-methoxyphenyl)pyrazole |
| J | 5-amino-4-cyano-1-(3-cyano-2,4 -dichlorophenyl)pyrazole |
| K | 5-amino-1-(4-bromo-2,3-dichlorophenyl)-4-cyanopyrazole |
| L | 5-amino-4-cyano-1-(2,3-dichloro-4-methylphenyl)pyrazole |
| M | 5-amino-4-cyano-1-(4-bromo-2-chloro-3-methylphenyl)pyrazole |
| N | 5-amino-4-cyano-1-(2-chloro-3,4-dimethylphenyl)pyrazole |
| P | 5-amino-4-cyano-1-(2-chloro-3-cyano-4-methylphenyl)pyrazole |
| Q | 5-amino-1-(3-chloro-2,4-dibromophenyl)-4-cyanopyrazole |
| R | 5-amino-1-(3-chloro-2,4-dimethylphenyl)-4-cyanopyrazole |
| S | 5-amino-1-(2-bromo-4-chloro-3-methylphenyl)-4-cyanopyrazole |
| T | 5-amino-1-(4-chloro-2,3-dimethylphenyl)-4-cyanopyrazole |
| U | 5-amino-1-(4-chloro-3-cyano-2-methylphenyl)-4-cyanopyrazole |
| V | 5-amino-4-cyano-1-(2,4,5-trichlorophenyl)pyrazole |
| X | 5-amino-4-cyano-1-(2,3,4,5-tetrachlorophenyl)pyrazole |
| Y | 5-amino-4-cyano-1-(2,3,4,6-tetrafluorophenyl)pyrazole |
| Z | 5-amino-4-cyano-1-pentachlorophenylpyrazole |
| AA | 5-amino-4-cyano-1-pentafluorophenylpyrazole |
| BB | 5-amino-4-cyano-1-(4-trifluoromethylphenyl)pyrazole |
| CC | 5-amino-4-cyano-1-(3-chloro-2,4-difluorophenyl)pyrazole |
| DD | 5-amino-4-N-methylcarbonamido-1-(2,3,4-trichlorophenyl)pyrazole |
| EE | 5-amino-4-N-ethylcarbonamido-1-(2,3,4-trichlorophenyl)pyrazole |

The letters of the alphabet A to H, J to N, P to V and X to EE are assigned to the above compounds for identification and easy reference hereafter in the present specification.

Particularly preferred compounds according to the present invention are, referring to the identification by letters of the alphabet indicated above, Compound C, and more especially Compounds D to H, J to N and P to U and, in particular, Compound A.

Accordingly, a feature of the present invention is a method for controlling the growth of weeds (i.e. undesired vegetation) at a locus which comprises applying to the locus a herbicidally effective amount of at least one N-phenylpyrazole derivative of general formula III. For this purpose, the N-phenylpyrazole derivatives are normally used in the form of herbicidal compositions (i.e. in association with compatible diluents or carriers suitable for use in herbicidal compositions), for example as hereinafter described.

The compounds of general formula III show herbicidal activity against dicotyledonous (i.e. broad-leafed) and monocotyledonous (e.g. grass) weeds by pre- and/or post-emergence application.

By the term "pre-emergence application" is meant application to the soil in which the weed seeds or seedlings are present before emergence of the weeds above the surface of the soil. By the term "post-emergence application" is meant application to the aerial or exposed portions of the weeds which have emerged above the surface of the soil. For example, the compounds of general formula III may be used to control the growth of broad-leafed weeds, for example, *Aethusa cynapium, Abutilon theophrasti, Amaranthus retroflexus, Amsinckia intermedia, Anagallis arvensis, Anthemis arvensis, Atriplex patula, Bidens Pilosa, Brassica nigra, Capsella bursa-pastoris, Chenopodium album, Chrysanthemum segetum, Cirsium arvense, Datura stramonium, Desmodium tortuosum, Emex australis, Euphorbia helioscopia, Fumaria officinalis, Galeopsis tetrahit, Galium aparine, Geranium dissectum, Ipomea purpurea, Lamium purpureum, Lapsana communis, Matricaria inodora, Monochoria vaginalis, Papaver rhoeas, Physalis longifolia, Plantago lanceolata, Polygonum* spp., (e.g. *Polygonum aviculare, Polygonum convolvulus* and *Polygonum persicaria*), *Portulaca oleracea, Raphanus raphanistrum, Rotala indica, Rumex obtusifolius, Saponaria vaccaria, Scandix pecten-veneris, Senecio vulgaris, Sesbania florida, Sida spinosa, Silene alba, Sinapis arvensis, Solanum nigrum, Sonchus arvensis, Spergula arvensis, Stellaria media, Thlaspi arvense, Tribulus terrestria, Urtica urens, Veronica hederifolia, Veronica persica, Viola arvensis* and *Xanthium strumarium,* and grass weeds, for example, *Alopecurus myosuroides, Apera spica-venti, Agrostis stolonifera, Avena fatua, Avena ludoviciana, Brachiaria* spp., *Bromus sterilis, Bromus techtorum, Cenchrus* spp., *Cynodon dactylon, Digitaria sanguinalis, Echinochloa crus-galli, Eleusine indica, Setaria viridis* and *Sorghum halepense* and sedges, for example *Cyperus esculentus, Cyperus iria* and *Cyperus rotundus,* and *Eleocharis acicularis.*

The amounts of compounds of general formula III applied vary with the nature of the weeds, the compositions used, the time of application, the climatic and edaphic conditions and (when used to control the growth of weeds in crop-growing areas) the nature of the crops. When applied to a crop-growing area, the rate of application should be sufficient to control the growth of weeds without causing substantial permanent damage to the crop. In general, taking these factors into account, application rates between 0.1 kg and 20 kg of active material per hectare give good results. However, it is to be understood that higher or lower application rates may be used, depending upon the particular problem of weed control encountered.

The compounds of general formula III may be used to control selectively the growth of weeds, for example to control the growth of those species hereinbefore mentioned, by pre- or post-emergence application in a directional or non-directional fashion, e.g. by directional or non-directional spraying, to a locus of weed infestation which is an area used, or to be used, for growing crops, for example cereals, e.g. wheat, barley, oats, maize and rice, soya beans, field and dwarf beans, peas, lucerne, cotton, peanuts, flax, onions, carrots, cabbage, oilseed rape, sunflower, sugar beet, and permanent or sown grassland before or after sowing of the crop or before or after emergence of the crop. For the selective control of weeds at a locus of weed infestation which is an area used, or to be used, for the growing of crops, e.g. the crops hereinbefore mentioned, application rates between 0.25 kg and 8.0 kg, and preferably between 0.5 kg and 2.0 kg, more especially of the preferred compounds of general formula III hereinbefore specified, of active material per hectare are particularly suitable. More particularly, the compounds of general formula III may be used to control selectively the growth of broad leafed weeds, for example to control the growth of those broad leafed species hereinbefore mentioned, by pre- or, more especially, post-emergence application in a non-directional fashion, e.g. by non-directional spraying, to an area used for growing cereal crops before or after emergence of both the crop and weeds.

For this purpose, i.e. the selective control of broad leafed weeds by pre- or post-emergence application to an area used for growing cereal crops, application rates between 0.25 and 8.0 kg, and preferably between 0.5 kg and 2.0 kg, more especially of the preferred compounds of general formula III hereinbefore specified, of active material per hectare are particularly suitable.

The compounds of general formula III may also be used to control the growth of weeds, especially those indicated above, by pre- or post-emergence application in established orchards and other tree-growing areas, for example forests, woods and parks, and plantations, e.g. sugar cane, oil palm and rubber plantations. For this purpose they may be applied in a directional or non-directional fashion (e.g. by directional or non-directional spraying) to the weeds or to the soil in which they are expected to appear, before or after planting of the trees or plantations at application rates between 0.25 kg and 10.0 kg, and preferably between 1.0 kg and 4.0 kg, more especially of the preferred compounds of general formula III hereinbefore specified, of active material per hectare.

The compounds of general formula III may also be used to control the growth of weeds, especially those indicated above, at loci which are not crop-growing areas but in which the control of weeds is nevertheless desirable. Examples of such non-crop-growing areas include airfields, industrial sites, railways, roadside verges, the verges of rivers, irrigation and other waterways, scrublands and fallow or uncultivated land, in particular where it is desired to control the growth of weeds in order to reduce fire risks. When used for such purposes in which a total herbicidal effect is frequently desired, the active compounds are normally applied at dosage rates higher than those used in crop-growing areas as hereinbefore described. The precise dosage will depend upon the nature of the vegetation treated and the effect sought. Pre- or post-emergence application, and preferably pre-emergence application, in a directional or

non-directional fashion (e.g. by directional or non-directional spraying) at application rates between 5.0 kg and 20.0 kg, and preferably between 10.0 and 20.0 kg, more especially of the preferred compounds of general formula III hereinbefore specified, of active material per hectare are particularly suitable for this purpose.

When used to control the growth of weeds by pre-emergence application, the compounds of general formula III may be incorporated into the soil in which the weeds are expected to emerge. It will be appreciated that when the compounds of general formula III are used to control the growth of weeds by post-emergence application, i.e. by application to the aerial or exposed portions of emerged weeds, the compounds of general formula III will also normally come into contact with the soil and may also then exercise a pre-emergence control on later-germinating weeds in the soil.

Where especially prolonged weed control is required, the application of the compounds of general formula III may be repeated if required.

According to a further feature of the present invention, there are provided compositions suitable for herbicidal use comprising one or more of the N-phenylpyrazole derivatives of general formula III in association with, and preferably homogeneously dispersed in, one or more compatible herbicidally-acceptable diluents or carriers (i.e. diluents or carriers of the type generally accepted in the art as being suitable for use in herbicidal compositions and which are compatible with compounds of general formula III). The term "homogeneously dispersed" is used to include compositions in which the compounds of general formula III are dissolved in the other components. The term "herbicidal compositions" is used in a broad sense to include not only compositions which are ready for use as herbicides but also concentrates which must be diluted before use. Preferably, the compositions contain from 0.05 to 90% by weight of one or more compounds of general formula III.

The herbicidal compositions may contain both a diluent or carrier and a surface-active (e.g. wetting, dispersing, or emulsifying) agent. Surface-active agents which may be present in herbicidal compositions of the present invention may be of the ionic or nonionic types, for example sulphoricinoleates, quaternary ammonium derivatives, products based on condensates of ethylene oxide with nonyl- or octylphenols, or carboxylic acid esters of anhydrosorbitols which have been rendered soluble by etherification of the free hydroxy groups by condensation with ethylene oxide, alkali and alkaline earth metal salts of sulphuric acid esters and sulphonic acids such as dinonyl- and dioctyl-sodium sulphonosuccinates and alkali and alkaline earth metal salts of high molecular weight sulphonic acid derivatives such as sodium and calcium lignosulphonates.

Suitably, herbicidal compositions according to the present invention may comprise from 0.05% to 10% of surface-active agent but, if desired, herbicidal compositions according to the present invention may comprise higher proportions of surface-active agent, for example up to 15% in liquid emulsifiable suspension concentrates and up to 25% in liquid water soluble concentrates.

Examples of suitable solid diluents or carriers are aluminium silicate, talc, calcined magnesia, kieselguhr, tricalcium phosphate, powdered cork, adsorbent carbon black and clays such as kaolin and bentonite. The solid compositions (which may take the form of dusts, granules or wettable powders) are preferably prepared by grinding the compounds of general formula III with solid diluents or by impregnating the solid diluents or carriers with solutions of the compounds of general formula III in volatile solvents, evaporating the solvents and, if necessary, grinding the products so as to obtain powders. Granular formulations may be prepared by absorbing the compounds of general formula III (dissolved in volatile solvents) onto the solid diluents or carriers in granular form and evaporating the solvents, or by granulating compositions in powder form obtained as described above. Solid herbicidal compositions, particularly wettable powders, may contain wetting or dispersing agent (for example of the types described above), which may also, when solid, serve as diluents or carriers.

Liquid compositions according to the invention may take the form of aqueous, organic or aqueous-organic solutions, suspensions and emulsions which may incorporate a surface-active agent. Suitable liquid diluents for incorporation in the liquid compositions include water, acetophenone, cyclohexanone, isophorone, toluene, xylene and mineral, animal and vegetable oils (and mixtures of these diluents). Surface-active agents, which may be present in the liquid compositions, may be ionic or non-ionic (for example of the types described above) and may, when liquid, also serve as diluents or carriers.

Wettable powders and liquid compositions in the form of concentrates may be diluted with water or other suitable diluents, for example mineral or vegetable oils, particularly in the case of liquid concentrates in which the diluent or carrier is an oil, to give compositions ready for use. When desired, liquid compositions of the compound of general formula III may be used in the form of self-emulsifying concentrates containing the active substances dissolved in the emulsifying agents or in solvents containing emulsifying agents compatible with the active substances, the simple addition of water to such concentrates producing compositions ready for use.

Liquid concentrates in which the diluent or carrier is an oil may be used without further dilution using the electrostatic spray technique.

Herbicidal compositions according to the present invention may also contain, if desired, conventional adjuvants such as adhesives, protective colloids, thickeners, penetrating agents, stabilisers, sequestering agents, anti-caking agents, colouring agents and corrosion inhibitors. These adjuvants may also serve as carriers or diluents.

Preferred herbicidal compositions according to the present invention are aqueous suspension concentrates which comprise from 10 to 70% w/v of one or more compounds of general formula III, from 2 to 10% w/v of surface-active agent, from 0.1 to 5% w/v of thickener and from 15 to 87.9% by volume of water; wettable powders which comprise from 10 to 90% w/w of one or more compounds of general formula III, from 2 to 10% w/w of surface-active agent and from 10 to 88% w/w of solid diluent or carrier; liquid water soluble concentrates which comprise from 10 to 30% w/v of one or more compounds of general formula III, from 5 to 25% w/v of surface-active agent and from 45 to 85% by volume of water-miscible solvent, e.g. dimethylformamide; liquid emulsifiable suspension concentrates which comprise from 10 to 70% w/v of one or more compounds of general formula III, from 5 to 15% w/v of surface active agent, from 0,1 to 5% w/v of thickener and from 10 to 84.9% by volume of organic solvent, and granules which comprise from 2 to 10% w/w of one or more compounds of general formula III, from 0.5 to 2% w/w of surface-active agent and from 88 to 97.5% w/w of granular carrier.

Herbicidal compositions according to the present invention may also comprise the compounds of general formula III in association with, and preferably homogeneously dispersed in, one or more other pesticidally active compounds and, if desired, one or more compatible pesticidally acceptable diluents or carriers, surface-active agents and conventional adjuvants as hereinbefore described. Examples of other pesticidally active compounds which may be included in, or used in conjunction with, the herbicidal compositions of the present invention include herbicides, for example to increase the range of weed species controlled, for example alachlor [α-chloro-2,6-diethyl-N-(methoxymethyl)acetanilide], asulam [methyl(4-aminobenzenesulphonyl)carbamate], alloxydim Na [sodium salt of 2-(1-allyloxyamino-butylidene)-5,5-dimethyl-4-methoxycarbonylcyclohexane-1,3-dione], atrazine [2-chloro-4-ethylamino-6-isopropylamino-1,3,5-triazine], barban [4-chlorobut-2-ynyl N-(3-chlorophenyl)carbamate], benzoylprop-ethyl [ethyl N-benzoyl-N-(3,4-dichlorophenyl-2-aminopropionate], bromoxynil [3,5-dibromo-4-hydroxy-benzonitrile], butachlor [N-(butoxymethyl)-α-chloro-2,6-diethylacetanilide], butylate [S-ethyl N,N-diiso-butyl(thiocarbamate)], carbetamide [D-N-ethyl-2-(phenylcarbamoyloxy)propionamide], chlorfenprop-methyl [methyl 2-chloro-3-(4-chlorophenyl)propionate], chlorpropham [isopropyl N-(3-chlorophenyl)-carbamate], chlortoluron [N'-(3-chloro-4-methylphenyl)-N,N-dimethylurea], cyanazine [2-chloro-4-(1-cyano-1-methylethylamino)-6-ethylamino-1,3,5-triazine], cycloate [N'-cyclohexyl-N-ethyl-S-ethyl(thiocarbamate)], 2,4-D [2,4-dichlorophenoxyacetic acid], dalapon [2,2-dichloropropionic acid], 2,4-DB [4-(2,4-dichlorophenoxy)butyric acid], desmedipham [3-(ethoxycarbonylamino)phenyl N-phenyl-carbamate], diallate [S-2,3-dichloroallyl-N,N-di-isopropyl(thiocarbamate)], dicamba [3,6-dichloro-2-methoxybenzoic acid], dichlorprop [(±)-2-(2,4-dichlorophenoxy)propionic acid], difenzoquat [1,2-dimethyl-3,5-diphenyl-pyrazolium salts], dimefuron {4-[2-chloro-4-(3,3-dimethylureido)phenyl]-2-t-butyl-1,3,4-oxadiazolin-5-one}, dinitramine [$N^1$,$N^1$-diethyl-2,6-dinitro-4-trifluoromethyl-*m*-phenylenediamine], diuron [N'-(3,4-dichlorophenyl)-N,N-dimethylurea], EPTC [S-ethyl N,N-dipropyl(thiocarbamate)], ethofumesate [2-ethoxy-2,3-dihydro-3,3-dimethylbenzofuran-5-yl methylsulphonate], flampropisopropyl [isopropyl (±)-2-(N-benzoyl-3-chloro-4-fluoroanilino)propionate], flampropmethyl [methyl (±)-2-(N-benzoyl-3-chloro-4-fluoroanilino)-propionate], fluometuron [N'-(3-trifluoromethylphenyl)-N,N-dimethylurea], ioxynil [4-hydroxy-3,5-di-iodo-benzonitrile], isoproturon [N'-(4-isopropylphenyl)-N,N-dimethylurea], linuron [N-(3,4-dichlorophenyl)-N-methoxy-N-methylurea], MCPA [4-chloro-2-methylphenoxyacetic acid], MCPB [4-(4-chloro-2-methyl-phenoxy)butyric acid], mecoprop [(±)-2-(4-chloro-2-methylphenoxy)propionic acid], metamitron [4-amino-3-methyl-6-phenyl-1,2,4-triazin-5(4H)-one], methabenzthiazuron [N-(benzothiazol-2-yl)-N,N'-dimethylurea], metribuzin [4-amino-6-t-butyl-3-(methylthio)-1,2,4-triazin-5(4H)-one], molinate [S-ethyl N,N-hexamethylene(thiocarbamate)], oxadiazon [3-(2,4-dichloro-5-isopropoxyphenyl)-5-t-butyl-1,3,4-oxadiazolin-2-one], paraquat [1,1'-dimethyl-4,4'-bipyridylium salts], pebulate [S-propyl N-butyl-N-ethyl(thiocarbamate)], phenmedipham [3-(methoxycarbonylamino)phenyl N-(3-methylphenyl)carbamate], prometryne [4,6-bisisopropylamino-2-methylthio-1,3,5-triazine], propachlor [α-chlor-N-isopropylacetanilide], propanil [N-(3,4-dichlorophenyl)propionamide], propham [isopropyl N-phenylcarbamate], pyrazone [5-amino-4-chloro-2-phenylpyridazin-3(2H)-one], simazine [2-chloro-4,6-bisethylamino-1,3,5-triazine], TCA (trichloroacetic acid), thiobencarb [S-(4-chlorobenzyl)-N,N-diethylthiolcarbamate], tri-allate [S-2,3,3-trichloroallyl N,N-di-isopropyl(thiocarbamate)] and trifluralin [2,6-dinitro-N,N-dipropyl-4-trifluoromethylaniline]- insecticides, e.g. carbaryl [naphth-1-yl N-methylcarbamate]; synthetic pyrethroids, e.g. permethrin and cypermethrin; and fungicides, e.g. 2,6-dimethyl-4-tridecyl-morpholine, methyl N-(1-butylcarbamoyl-benz-imidazol-2-yl)carbamate, 1,2-bis-(3-methoxycarbonyl-2-thioureido)benzene, isopropyl 1-carbamoyl-3-(3,5-dichlorophenyl)hydantoin and 1-(4-chloro-phenoxy)-3,3-dimethyl-1-(1,2,4-triazol-1-yl)-butan-2-one. Other biologically active materials which may be included in, or used in conjunction with, the herbicidal compositions of the present invention are plant growth regulators, e.g succinamic acid, (2-chloroethyl)trimethylammonium chloride and 2-chloroethane phosphonic acid; or fertilizers, e.g. containing nitrogen, potassium and phosphorus and trace elements known to be essential to successful plant life, e.g. iron, magnesium, zinc, manganese, cobalt and copper.

Pesticidally active compounds and other biologically active materials which may be included in, or used in conjunction with, the herbicidal compositions of the present invention, for example those hereinbefore mentioned, and which are acids, may, if desired, be utilized in the form of conventional derivatives, for example alkali metal and amine salts and esters.

When the diluent or carrier for the N-phenylpyrazole derivative of general formula III is water or a

common organic solvent (e.g. toluene or xylene) and the active ingredient of the herbicidal composition is either of the previously known compounds 5-amino-4-cyano-1-(2,4-dichlorophenyl)pyrazole and 5-amino-4-cyano-1-(4-chloro-2-methylphenyl)pyrazole (i.e. the compounds of general formula III wherein $R^{10}$ represents the cyano radical and $R^5$ and $R^6$ each represent a chlorine atom and $R^7$, $R^8$ and $R^9$ each represent a hydrogen atom or $R^5$ represents the methyl radical, $R^6$ represents a chlorine atom and $R^7$, $R^8$ and $R^9$ each represent a hydrogen atom), another material should also be present, for example a surface-active agent, another pesticide, or a fertilizer, in order that the composition is other than an association of either of the two aforesaid pyrazole derivatives solely with water or a common organic solvent. The present invention does not comprise an association of the known compound 5-amino-4-cyano-1-(2,4,5-trichlorophenyl)-pyrazole or 5-amino-4-cyano-1-(2,4-dinitrophenyl)pyrazole (i.e. the compounds of general formula III wherein (i) $R^5$, $R^6$ and $R^8$ each represent a chlorine atom and $R^7$ and $R^9$ each represent a hydrogen atom, (ii) $R^5$ and $R^6$ each represent a nitro radical and $R^7$, $R^8$ and $R^9$ each represent a hydrogen atom, and $R^{10}$ in both instances represents a cyano radical) solely with water or a common organic solvent.

An article of manufacture may comprise at least one of the N-phenylpyrazole derivatives of general formula III or, as is preferred, a herbicidal composition as hereinbefore described, and preferably a herbicidal concentrate which must be diluted before use, comprising at least one of the N-phenylpyrazole derivatives of general formula III within a container for the aforesaid derivative or derivatives of general formula III, or a said herbicidal composition, and instructions physically associated with the aforesaid container setting out the manner in which the aforesaid derivative or derivatives of general formula III or herbicidal composition contained therein is to be used to control the growth of weeds. The containers will normally be of the types conventionally used for the storage of chemical substances which are solid at normal ambient temperatures and herbicidal compositions particularly in the form of concentrates, for example cans and drums of metal, which may be internally-lacquered, and plastics materials, bottles of glass and plastics materials and, when the contents of the container is a solid, for example granular, herbicidal compositions, boxes, for example of cardboard, plastics materials and metal, or sacks. The containers will normally be of sufficient capacity to contain amounts of the N-phenylpyrazole derivative or herbicidal compositions sufficient to treat at least one acre of ground to control the growth of weeds therein but will not exceed a size which is convenient for conventional methods of handling. The instructions will be physically associated with the container, for example by being printed directly thereon or on a label or tag affixed thereto. The directions will normally indicate that the contents of the container, after dilution if necessary, are to be applied to control the growth of weeds at rates of application between 0.1 kg and 20 kg of active material per hectare in the manner and for the purposes hereinbefore described.

The following Examples illustrate herbicidal compositions according to the present invention.

In the following, "Celite", "Aerosil", "Ethylan", "Sopropon", "Rhodigel", "Bentone", "Aromasol", "Arylan", and "Darvan", are Trade Marks.

## Example 1

5-Amino-4-cyano-1-pentafluorophenylpyrazole was formulated as a water soluble concentrate containing

| | |
|---|---|
| 5-amino-4-cyano-1-pentafluorophenylpyrazole | 10% w/v (weight/volume) |
| Ethylan KEO (nonylphenyl/ethylene oxide condensate containing 9—10 moles of ethylene oxide per mol of phenol) | 10% w/v |
| Dimethylformamide | to 100% by volume |

by dissolving the Ethylan KEO in a portion of dimethylformamide and then adding the active ingredient with heating and stirring until dissolved. The resulting solution was then made up to 100% by volume by adding the rest of the dimethylformamide.

20 Litres of the above formulation may be dissolved in 200 litres of water and sprayed post-emergence onto 1 hectare of oilseed rape to control *Amaranthus retroflexus, Setaria viridis, Polygonum lapathifolium, Abutilon theophrasti* and *Solanum nigrum*.

The 5-amino-4-cyano-1-pentafluorophenylpyrazole may, if desired, be replaced in the above water soluble concentrate by any other compound of general formula III.

## Example 2

A wettable powder was formed from:—

| | |
|---|---|
| 5-amino-4-cyano-1-(2,3,4-trichlorophenyl)pyrazole | 50% w/w (weight/weight) |

| | |
|---|---|
| Ethylan BCP (a nonylphenol/ethylene oxide condensate containing 9 moles of ethylene oxide per mol of phenol) | 5% w/w |
| Aerosil (silicon dioxide of microfine particle size) | 5% w/w |
| Celite PF (synthetic magnesium silicate carrier) | 40% w/w |

by adsorbing the Ethylan BCP onto the Aerosil, mixing with the other ingredients and grinding the mixture in a hammer-mill to give a wettable powder which may be diluted with water and applied at an application rate of 4 kg of wettable powder in 300 litres of spray fluid per hectare to control the growth of *Galium aparine, Veronica persica, Viola arvensis* and *Galeopsis tetrahit* by post-emergence application in an emerged crop of winter wheat.

Similar wettable powders may be prepared as described above by replacing the 5-amino-4-cyano-1-(2,3,4-trichlorophenyl)pyrazole by other compounds of general formula III.

Example 3

An aqueous suspension concentrate was formed from:—

| | |
|---|---|
| 5-amino-4-cyano-1-(2,3,4-trichlorophenyl)pyrazole | 50% w/v |
| Ethylan BCP | 1.0% w/v |
| Sopropon T36 (sodium salt of polycarboxylic acid) | 0.2% w/v |
| Ethylene glycol | 5% w/v |
| Rhodigel 23 (polysaccharide xanthan gum thickener) | 0.15% w/v |
| distilled water | to 100% by volume |

by intimately mixing the ingredients and grinding in a ball-mill for 24 hours. The concentrate thus obtained may be dispersed in water and applied at an application rate of 4 kg of aqueous suspension concentrate in 300 litres of spray fluid per hectare to control the growth of *Galium aparine, Veronica persica, Viola arvensis* and *Galeopsis tetrahit* by post-emergence application in an emerged crop of winter barley.

Similar aqueous suspension concentrates may be prepared as described above by replacing the 5-amino-4-cyano-1-(2,3,4-trichlorophenyl)pyrazole by other compounds of general formula III

Example 4

An emulsifiable suspension concentrate was formed from:—

| | |
|---|---|
| 5-amino-4-cyano-1-(2,3,4-trichlorophenyl)pyrazole | 50% w/v |
| Ethylan TU (a nonyl phenol/ethylene oxide condensate containing 10 moles of ethylene oxide per mol of phenol) | 10% w/v |
| Bentone 38 (an organic derivative of special magnesium montmorillonite thickener) | 0.5% w/v |
| Aromasol H (an aromatic solvent consisting predominantly of isomeric trimethylbenzenes) | to 100% by volume |

by intimately mixing the ingredients and grinding in a ball-mill for 24 hours. The emulsifiable suspension concentrate thus obtained may be diluted with water and applied at an application rate of 3 kg of emulsifiable suspension concentrate in 100 litres of spray fluid per hectare to control the growth of *Setaria viridis, Polygonum convolvulus,* and *Chenopodium album* by post-emergence application in an emerged crop of spring-sown wheat.

Similar emulsifiable suspension concentrates may be prepared as described above by replacing the 5-amino-4-cyano-1-(2,3,4-trichlorophenyl)pyrazole by other compounds of general formula III.

Example 5

Granules were formed from:—

| | |
|---|---|
| 5-amino-4-cyano-1-(2,3,4-trichlorophenyl)pyrazole | 5% w/w |
| Ethylan BCP | 1% w/w |
| Oleic acid | 1% w/w |
| Aromasol H | 12% w/w |
| 30/60 Attapulgite granules (sorptive silica clay) | 81% w/w |

by mixing the phenylpyrazole, Ethylan BCP, oleic acid and Aromasol H and spraying the mixture onto the Attapulgite granules. The granules thus obtained may be applied at an application rate of 50 kg of granules per hectare to control the growth of *Echinochloa crus-galli, Eleocharis acicularis* and *Monochoria vaginalis* by pre-emergence application or application to seedling weeds in a crop of transplanted paddy rice.

Similar granules may be prepared as described above by replacing the 5-amino-4-cyano-1-(2,3,4-trichlorophenyl)pyrazole by other compounds of general formula III.

Example 6

A water soluble concentrate was formed from:—

| | |
|---|---|
| 5-amino-4-cyano-1-(2,3,4-trichlorophenyl)pyrazole | 10% w/v |
| Ethylan KEO | 10% w/v |
| Dimethylformamide | to 100% by volume |

by dissolving the Ethylan KEO in a portion of dimethylformamide and then adding the pyrazole derivative with heating and stirring until dissolved. The resulting solution was then made up to 100% by volume with dimethylformamide by adding the rest of the dimethylformamide. The water soluble concentrate thus obtained may be diluted with water and applied at an application rate of 10 litres of water soluble concentrate in 200 to 2000 litres of spray fluid per hectare to control the growth of *Galium aparine, Veronica persica, Viola arvensis* and *Galeopsis tetrahit* by post-emergence application in an emerged crop of winter wheat at the tillering growth stage.

Example 7

A wettable powder was formed from:—

| | |
|---|---|
| 5-amino-4-cyano-1-(2,3,4-trichlorophenyl)pyrazole | 90% w/w |
| Arylan S (sodium dodecyl benzene sulphonate) | 2% w/w |
| Darvan No. 2 (sodium lignosulphate) | 5% w/w |
| Celite PF | 3% w/w |

by mixing the ingredients and grinding the mixture in a hammer-mill to give a wettable powder which may be diluted with water and applied at an application rate of 2 kg of wettable powder in 300 litres of spray fluid per hectare to control the growth of *Galium aparine, Veronica persica, Viola arvensis* and *Galeopsis tetrahit* by post-emergence application in an emerged crop of winter wheat.

Similar wettable powders may be prepared as described above by replacing the 5-amino-4-cyano-1-(2,3,4-trichlorophenyl)pyrazole by other compounds of general formula III.

Example 8

A wettable powder containing 50% w/w of 5-amino-4-cyano-1-(2,3,4-trichlorophenyl)pyrazole, prepared as hereinbefore described in Example 2, may be diluted with water and applied at an application rate of 0.5 kg of wettable powder in 300 litres of spray fluid per hectare to control the growth of *Abutilon theophrasti* and *Polygonum lapathifolium* by post-emergence application at the early seedling growth stage of these weeds in a crop of spring wheat.

Example 9

A wettable powder containing 50% w/w of 5-amino-4-cyano-1-(2,3,4-trichlorophenyl)pyrazole, prepared as described in Example 2, may be diluted with water and applied at an application rate of 40 kg of

wettable powder in 600 litres of spray fluid per hectare to produce a total herbicidal effect on vegetation at a locus which is not a crop-growing area.

In experiments on herbicidal activity carried out on representative compounds of general formula III, the closely related compound 1-phenyl-4-cyano-5-aminopyrazole specifically disclosed in Japanese Patent Application No. 29598/1963 (test compound CC1) and the closely related compound 5-amino-4-cyano-1-(2,3,4-trichlorophenyl)-3-methylpyrazole (test compound CC2), the following results have been obtained:—

TEST METHODS

(1) Weed Control Test

(a) *General*

The test compounds A to H, J to N, P to V and X to EE, CC1 and CC2 (as hereinbefore identified) were dissolved in acetone. Application was from a standard laboratory herbicide sprayer using a flat fan jet travelling at 1.6 m.p.h. (2.6 km/hour) and delivering the equivalent of 530 litres of spray fluid per hectare, the spray pressure being 2.81 kg/cm$^2$ (40 pounds/inch$^2$). The solutions of test compounds A to H, J, K, M, N and Q to V and X to EE, CC1 and CC2 were prepared by dissolving 0.513 g of test compound in acetone and making up with more acetone to 34 ml (1.5% w/v), equivalent to an application rate of 8 kg of test compound per hectare. Solutions equivalent to 4, 2, 1, 0.5, 0.25 and 0.125 kilogrammes per hectare (kg/ha) were prepared from these solutions by serial dilution with acetone, except for test compounds C, AA, BB, DD, EE and CC1 for which solutions equivalent to 8, 4, 2, 1 and 0.5 kg/ha were prepared. The solutions of test compounds L and P were similarly prepared but using 0.128 g of test compound to give solutions equivalent to application rates of 2, 1, 0.5, 0.25 and 0.125 kg/ha.

(b) *Weed Control: Pre-emergence application*

Weed seeds were sown on the surface of John Innes No. 1 potting compost (7 parts by volume of sterilized loam, 3 parts by volume of peat and 2 parts by volume of fine grit) in 9 cm diameter bitumenised paper pots. The quantities of seeds per pot were as follows:—

| Weed species | Approximate number seeds/pot |
|---|---|
| (i) *Broad leafed weeds* | |
| *Sinapis arvensis* | 30—40 |
| *Polygonum lapathifolium* | 30—40 |
| *Stellaria media* | 30—40 |
| (ii) *Grass weeds* | |
| *Avena fatua* | 15—20 |
| *Alopecurus myosuroides* | 30—40 |
| *Echinochloa crus-galli* | 20—30 |

The test compounds were applied to the uncovered seeds as described in (1) (a) above at dose rates of 0.125 to 8 kg/ha, except for test compounds L and P, which were applied at dose rates of 0.125 to 2 kg/ha, and for test compounds C, AA, BB, DD, EE and CC1 which were applied at dose rates of 0.5 to 8 kg/ha, and the seeds were covered with 25 ml of sharp sand after spraying. A single pot of each weed species was allocated to each treatment, with unsprayed controls and controls sprayed with acetone alone. After treatment, the pots were kept in the greenhouse and were watered overhead. Visual assessment of weed control activity was made 19 to 28 days after spraying. The results were expressed as the minimum effective dose (MED) in kg/ha which gave 90% reduction in growth or kill of the weeds in comparison with plants in the control pots. The results obtained are presented below in Table I.

(c) *Weed Control: Post-emergence application*

Weed species were grown and then transplanted at the seedling stage into John Innes No. 1 potting compost in 9 cm diameter bitumenised paper pots, except for *Avena fatua*, which was sown directly in the test pot and not transplanted. The plants were then grown in the greenhouse until ready for spraying with the test compounds. The number of plants per pot and the growth stage of the plant at spraying were as follows:—

| Weed species | Number of plants/pot | Growth stages at spraying |
|---|---|---|
| (i) *Broad leafed weeds* | | |
| *Polygonum lapathifolium* | 5 | 1—1 1/2 pairs of leaves |
| *Stellaria media* | 5 | 4—6 leaves |
| *Abutilon theophrasti* | 3 | 2 pairs of leaves |
| (ii) *Grass weeds* | | |
| *Avena fatua* | 10 | 1 leaf |
| *Alopecurus myosuroides* | 5 | 1 1/2 leaves |
| *Echinochloa crus-galli* | 5 | 1—2 leaves |

The test compounds were applied to the plants as described in (1) (a) above at dose rates of from 0.125 to 8 kg/ha except for test compounds L and P, which were applied at dose rates of 0.125 to 2 kg/ha and for test compounds C, AA, BB, DD, EE and CC1 which were applied at dose rates of 0.5 to 8 kg/ha. A single pot of each weed species was allocated to each treatment, with unsprayed controls and controls sprayed with acetone alone. After spraying, the pots were watered overhead, commencing 24 hours after spraying. Assessment of the control of the growth of the weeds was made 19—28 days after spraying by recording the number of plants which had been killed and the reduction in growth. The results were expressed as the minimum effective dose (MED) in kg/ha which gave 90% reduction in growth or kill of the weeds in comparison with the plants in the control pots. The results obtained are presented below in Table II.

KEY TO WEED SPECIES

(a) *Grass Weeds:—*

Am = *Alopecurus myosuroides*
Af = *Avena fatua*
Ec = *Echinochloa crus-galli*

(b) *Broad-leaf Weeds*

Sm = *Stellaria media*
Pl = *Polygonum lapathifolium*
Sa = *Sinapis arvensis*
At = *Abutilon theophrasti*

TABLE I

| Test Compound | PRE-EMERGENCE MED (kg/ha) | | | | | |
|---|---|---|---|---|---|---|
| | Pl | Sa | Sm | Am | Af | Ec |
| A | 0.25 | 0.125 | 0.5–1 | 0.5 | 0.5–1 | 0.125–0.25 |
| B | 0.5–1 | 0.5 | >8 | 2 | 2–4 | 1–2 |
| C | 0.5–1 | 0.5–1 | 8 | 4 | 4 | 1 |
| D | 0.5–1 | 0.25–0.5 | 4–8 | 2 | 4 | 1 |
| E | 1 | 1–2 | 2–4 | 2–4 | 2–4 | 0.5–1 |
| F | 0.5–1 | 0.5–1 | 2 | 2–4 | 1–2 | 0.5–1 |
| G | 0.25–0.5 | 0.25–0.5 | 0.5 | 1–2 | 1–2 | 0.25–0.5 |
| H | 0.5–1 | 2 | 1 | 2 | 1–2 | 2 |
| J | 0.5 | 0.5–1 | 4–8 | 2 | 1–2 | 1 |
| K | 1 | 0.5 | 1–2 | 2–4 | 2–4 | 1 |
| L | 0.5 | 1 | >>2 | >2 | >2 | 1–2 |
| M | 1 | 0.5–1 | >8 | 4 | 4 | 0.5–1 |
| N | 0.125–0.25 | 0.25 | 8 | 4 | 2–4 | 0.25–0.5 |
| P | 1 | >>2 | NR | NR | NR | 2 |
| Q | 4 | 4 | 4 | 4–8 | 4–8 | 4–8 |
| R | 1–2 | 4–8 | >8 | 2–4 | 2–4 | 1–2 |
| S | 1–2 | 0.5–1 | 2–4 | 4 | 4 | 4 |
| T | 0.5–1 | 2–4 | 4–8 | 0.5–1 | 2–4 | 2–4 |
| U | 2 | 4–8 | 4–8 | 2–4 | >8 | 2 |
| V | 1 | 2 | 8 | 8 | 4–8 | 1 |
| X | 2 | 1–2 | 0.5 | 2–4 | 2–4 | 2 |
| Y | 0.5 | 1.0 | 4–8 | 2–4 | 2 | 0.5–1 |
| Z | 2–4 | 1–2 | >>8 | 2–4 | 4–8 | 1 |

TABLE I (Continued)

| Test Compound | PRE-EMERGENCE MED (kg/ha) | | | | | |
|---|---|---|---|---|---|---|
| | Pl | Sa | Sm | Am | Af | Ec |
| AA | 1–2 | 1.0 | 8 | 2–4 | 1–2 | 1 |
| BB | 1–2 | 4 | 4–8 | 2–4 | 4 | 1–2 |
| CC | 1 | 4 | >>8 | 4 | 4 | 1 |
| DD | 4 | 1 | 8 | >>8 | NR | >8 |
| EE | NR | 2–4 | NR | NR | NR | >>8 |
| CCl | 8 | >>8 | 8 | NR | NR | >>8 |
| CC2 | NR | NR | NR | NR | NR | NR |

TABLE II

| Test Compound | POST-EMERGENCE MED (kg/ha) | | | | | |
|---|---|---|---|---|---|---|
| | At | Pl | Sm | Am | Af | Ec |
| A | 0.125 | 0.125 | 1–2 | 4–8 | 4–8 | 2–4 |
| B | 0.125 | 0.5 | >8 | >>8 | >8 | 8 |
| C | <0.5 | <0.5 | >>8 | >8 | >8 | 4 |
| D | 0.125–0.25 | 0.125–0.25 | 2–4 | >8 | >>8 | >8 |
| E | <0.125 | <0.125 | 2 | >>8 | >8 | 8 |
| F | 0.125–0.25 | 0.25–0.5 | 0.5–1 | NR | >>8 | >8 |
| G | <0.125 | 0.25–0.5 | 4–8 | >>8 | >8 | 4 |
| H | 0.25 | 0.125–0.25 | 0.25 | >8 | 8 | 1–2 |
| J | <0.125 | <0.125 | NR | >8 | >8 | 1–2 |
| K | 0.25–0.5 | 0.125–0.25 | 2 | NR | >8 | >8 |
| L | <0.125 | 0.25–0.5 | >2 | >>2 | >>2 | 2 |
| M | 0.125–0.25 | 0.25–0.5 | >8 | NR | >>8 | >8 |
| N | 0.125–0.25 | 0.25–0.5 | NR | >>8 | >>8 | 8 |
| P | 0.125–0.25 | 0.5–1 | >>2 | >>2 | >>2 | NR |
| Q | 0.125–0.25 | 0.25 | 4–8 | >>8 | >>8 | >>8 |
| R | 0.125–0.25 | 0.25–0.5 | NR | >>8 | >>8 | >8 |
| S | 0.125–0.25 | 0.25–0.5 | 1–2 | NR | >>8 | >8 |
| T | 0.5–1 | 0.5–1 | NR | >>8 | >8 | >8 |
| U | 1–2 | 0.5–1 | >>8 | >>8 | >>8 | >8 |

TABLE II (Continued)

| Test Compound | POST-EMERGENCE MED (kg/ha) | | | | | |
|---|---|---|---|---|---|---|
| | At | Pl | Sm | Am | Af | Ec |
| V | 0.25–0.5 | 0.5 | >>8 | >>8 | >>8 | >8 |
| X | 0.5–1 | 0.5–1 | 2 | NR | NR | >8 |
| Y | 0.125–0.25 | 0.25–0.5 | >>8 | 8 | 4–8 | 2 |
| Z | 0.5–1 | 1–2 | NR | >8 | NR | >8 |
| AA | <0.5 | <0.5 | NR | 8 | 4 | 2–4 |
| BB | 1 | 0.5–1 | NR | >>8 | >>8 | 4–8 |
| CC | 2–4 | 2 | NR | >>8 | >8 | 4 |
| DD | 1–2 | >8 | NR | NR | NR | >>8 |
| EE | 4–8 | 8 | NR | NR | NR | >>8 |
| CC1 | >>8 | >>8 | NR | >>8 | >>8 | NR |
| CC2 | NR | NR | NR | NR | >>8 | NR |

The following symbols which appear in the above Tables have the following meanings:—

'>>' means = much greater than
'>' means = greater than
'<' means = less than
'NR' means = no reduction at any dose rate applied

The above experimental results clearly demonstrate the valuable herbicidal properties of the compound of general formula III and the surprising and unexpected superiority in herbicidal activity possessed by the compounds of general formula III in comparison with the closely related compound 1-phenyl-4-cyano-5-aminopyrazole disclosed in Japanese Patent Application No. 29598/63 and a closely related compound 5-amino-4-cyano-1-(2,3,4-trichlorophenyl)-3-methylpyrazole in which substitution of the 3-position of the pyrazole ring by alkyl (methyl) as taught in Japanese Patent Application No. 29598/63 is combined with substitution in the 1-position of the pyrazole ring by a substituted phenyl group (2,3,4-trichlorophenyl) found to confer high herbicidal activity in the compounds of general formula III.

According to a feature of the present invention, the new N-phenylpyrazole derivatives of general formula III, wherein $R^5$, $R^6$, $R^7$, $R^8$ and $R^9$ are as hereinbefore defined and $R^{10}$ represents a cyano radical, with the exclusion of 5-amino-4-cyano-1-(2,4-dichlorophenyl)pyrazole, 5-amino-4-cyano-1-(2,4,5-trichlorophenyl)pyrazole, 5-amino-4-cyano-1-(4-chloro-2-methylphenyl)pyrazole, 5-amino-4-cyano-1-(2,4-dinitrophenyl)pyrazole and 5-amino-4-cyano-1-(2,4,6-trichlorophenyl)pyrazole, are prepared by the process which comprises the cyclisation of a compound of the general formula:—

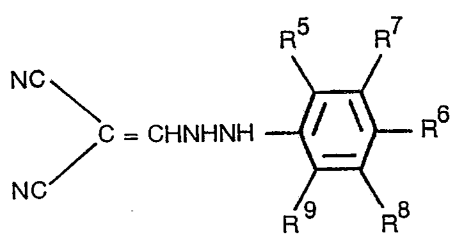

IV

wherein the various symbols are as hereinbefore defined with the provisos that (i) $R^5$ and $R^6$ are other than

chlorine atoms when $R^7$, $R^8$ and $R^9$ are hydrogen atoms, (ii) $R^5$ is other than the methyl radical when $R^6$ is a chlorine atom and $R^7$, $R^8$ and $R^9$ are hydrogen atoms, (iii) $R^5$ is other than a chlorine atom when $R^6$ and $R^8$ each represent a chlorine atom and $R^7$ and $R^9$ each represent a hydrogen atom, (iv) $R^5$ is other than a nitro radical when $R^6$ is a nitro radical and $R^7$, $R^8$ and $R^9$ each represent a hydrogen atom, and (v) $R^5$ is other than a chlorine atom when $R^6$ and $R^9$ each represent a chlorine atom and $R^7$ and $R^8$ each represent a hydrogen atom. Cyclisation may be effected in the presence of an inert organic solvent, for example an alkanol containing from 1 to 4 carbon atoms (e.g. ethanol), acetic acid or ethoxyethanol, at a temperature of from ambient temperature up to the reflux temperature of the reaction mixture.

Compounds of general formula IV may be prepared by the reaction of a compound of the general formula:—

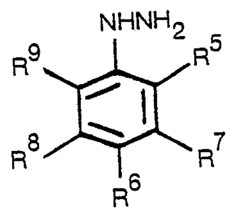

V

(wherein the various symbols are as hereinbefore defined with the provisos that (i) $R^5$ and $R^6$ are other than chlorine atoms when $R^7$, $R^8$ and $R^9$ are hydrogen atoms, (ii) $R^5$ is other than the methyl radical when $R^6$ is a chlorine atom and $R^7$, $R^8$ and $R^9$ are hydrogen atoms, (iii) $R^5$ is other than a chlorine atom when $R^6$ and $R^8$ each represent a chlorine atom and $R^7$ and $R^9$ each represent a hydrogen atom, (iv) $R^5$ is other than a nitro radical when $R^6$ is a nitro radical and $R^7$, $R^8$ and $R^9$ each represent a hydrogen atom, and (v) $R^5$ is other than a chlorine atom when $R^6$ and $R^9$ each represent a chlorine atom and $R^7$ and $R^8$ each represent a hydrogen atom) or an acid addition salt thereof (e.g. the hydrochloride) with a compound of the general formula;—

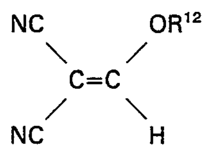

VI

wherein $R^{12}$ represents a straight- or branched-chain alkyl radical containing from 1 to 4 carbon atoms, preferably ethyl.

The reaction of a compound of general formula V with a compound of general formula VI may be effected in the presence of an inert organic solvent, for example an alkanol containing from 1 to 4 carbon atoms (e.g. ethanol), acetic acid or ethoxyethanol and at a temperature of from ambient temperature to the reflux temperature of the reaction mixture and optionally in the presence of an alkali metal (e.g. sodium or potassium), acetate, carbonate or bicarbonate. When an acid addition salt of the compound of general formula V is used, the reaction with the compound of general formula VI is effected in the presence of an alkali metal (e.g. sodium or potassium) acetate, carbonate or bicarbonate.

N-Phenylpyrazole derivatives of general formula III with the exclusion of 5-amino-4-cyano-1-(2,4-dichlorophenyl)pyrazole, 5-amino-4-cyano-1-(2,4,5-trichlorophenyl)pyrazole, 5-amino-4-cyano-(4-chloro-2-methylphenyl)pyrazole, 5-amino-4-cyano-1-(2,4-dinitrophenyl)pyrazole and 5-amino-4-cyano-1-(2,4,6-trichlorophenyl)pyrazole, wherein $R^5$, $R^6$, $R^7$, $R^8$ and $R^9$ are as hereinbefore defined and $R^{10}$ represents a cyano radical with the provisos that (i) $R^5$ and $R^6$ are other than chlorine atoms when $R^7$, $R^8$ and $R^9$ are hydrogen atoms, (ii) $R^5$ is other than the methyl radical when $R^6$ is a chlorine atom and $R^7$, $R^8$ and $R^9$ are hydrogen atoms, (iii) $R^5$ is other than a chlorine atom when $R^6$ and $R^8$ each represent a chlorine atom and $R^7$ and $R^9$ each represent a hydrogen atom, (iv) $R^5$ is other than a nitro radical when $R^6$ is a nitro radical and $R^7$, $R^8$ and $R^9$ each represent a hydrogen atom, and (v) $R^5$ is other than a chlorine atom when $R^6$ and $R^9$ each represent a chlorine atom and $R^7$ and $R^8$ each represent a hydrogen atom, may, according to another feature of the present invention, be prepared by reaction of a compound of general formula V with a compound of general formula VI as hereinbefore described without isolation of an intermediate compound of general formula IV from the reaction mixture. When the reaction of a compound of general formula V with a compound of general formula VI is effected in acetic acid, in the absence or presence of an alkali metal (e.g. sodium or potassium) acetate, the intermediate compound of general formula IV may separate from the reaction mixture, depending upon the solubility of the intermediate compound of general formula IV in the reaction medium, and may, if desired, be isolated before being cyclised as hereinbefore described to a compound of general formula III, preferably by heating in an inert organic solvent (e.g. ethoxyethanol) at the reflux temperature of the reaction mixture.

Isolated compounds of general formula IV have been found to exhibit herbicidal activities similar to those of the corresponding N-phenylpyrazole derivatives of general formula III into which they may be cyclised, and it is believed that the herbicidal activity of compounds of general formula IV results from their

cyclisation to compounds of general formula III.

According to a further feature of the present invention, the N-phenylpyrazole derivatives of general formula III, wherein $R^5$, $R^6$, $R^7$, $R^8$ and $R^9$ are as hereinbefore defined except for the primary amino and cyano radicals and $R^{10}$ represents a substituted carbamoyl group —$CONHR^{11}$ (wherein $R^{11}$ is as hereinbefore defined), are prepared by the selective alkylation of a compound of the general formula:—

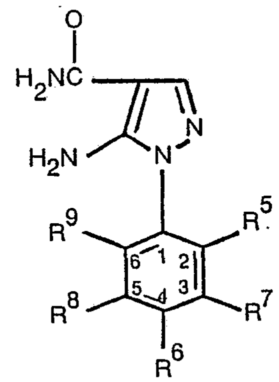

VII

(wherein $R^5$, $R^6$, $R^7$, $R^8$ and $R^9$ are as hereinbefore defined except for the primary amino and cyano radicals) with methyl iodide or ethyl iodide, in the presence of an inorganic base.

Compounds of general formula VII (wherein $R^5$, $R^6$, $R^7$, $R^8$ and $R^9$ are as hereinbefore defined, except for the primary amino and cyano radicals) may be prepared from the corresponding compounds of general formula III wherein $R^{10}$ represents a cyano radical by hydrolysis, for example by treatment with concentrated sulphuric acid.

According to a further feature of the present invention, N-phenylpyrazole derivatives of general formula III, wherein $R^5$, $R^6$, $R^7$, $R^8$ and $R^9$ are as hereinbefore defined and $R^{10}$ represents a substituted carbamoyl group —$CONHR^{11}$ (wherein $R^{11}$ is as hereinbefore defined), are prepared by reaction of a compound of the general formula:—

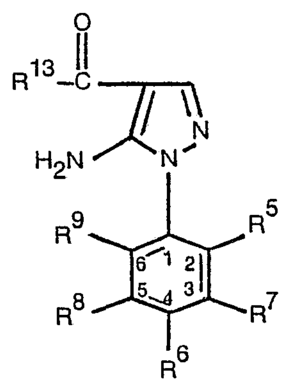

VIII

(wherein $R^5$, $R^6$, $R^7$, $R^8$ and $R^9$ are as hereinbefore defined, and $R^{13}$ represents a chlorine atom or a straight- or branched-chain alkoxy radical containing from 1 to 4 carbon atoms) with an amine of the general formula:—

$$H_2NR^{11} \qquad IX$$

wherein $R^{11}$ is as hereinbefore defined.

When $R^{13}$ represents a chlorine atom, reaction of a compound of general formula VIII with an amine of general formula IX is effected at a temperature between ambient temperature and the reflux temperature of the reaction mixture, in the presence of a solvent, which may be an inert organic solvent (e.g. acetone or toluene), or an excess of the amine of general formula IX if the boiling point of the latter is sufficiently high, and optionally in the presence of an acid-binding agent, e.g. sodium bicarbonate, potassium bicarbonate, triethylamine or an excess of the amine of general formula IX.

When $R^{13}$ represents an alkoxy radical, reaction of a compound of general formula VIII with an amine of general formula IX is effected in an inert organic solvent (e.g. ethanol) at a temperature between the reflux temperature of the reaction mixture and 200°C, if necessary at elevated pressure.

Compounds of general formula VIII wherein $R^{13}$ represents a chlorine atom may be prepared by the treatment of a compound of the general formula:—

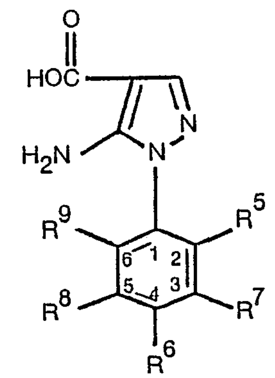

X

(wherein the various symbols are as hereinbefore defined) with thionyl chloride, optionally in an inert organic solvent, e.g. toluene or chloroform.

Compounds of general formula VIII wherein $R^{13}$ represents an alkoxy radical may be prepared by the cyclisation of a compound of the general formula:—

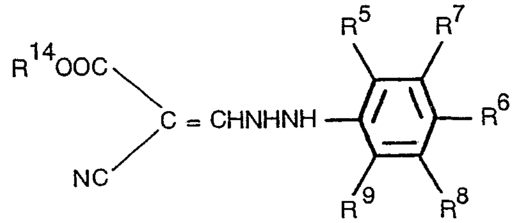

XI

wherein $R^5$, $R^6$, $R^7$, $R^8$ and $R^9$ are as hereinbefore defined and $R^{14}$ represents a straight- or branched-chain alkyl group containing from 1 to 4 carbon atoms. Cyclisation may be effected by heating the compound of general formula XI in an inert organic solvent (e.g. ethoxyethanol) at the reflux temperature of the reaction mixture.

Compounds of general formula VIII wherein $R^{13}$ represents an alkoxy radical may also be prepared by the esterification of a compound of general formula X by methods known *per se*, for example by reaction with the appropriate alcohol in the presence of an inorganic acid. Compounds of general formula X may be prepared by the hydrolysis under alkaline conditions, for example with potassium hydroxide, of the ester group $R^{13}$—CO— of a compound of general formula VIII, wherein $R^{13}$ represents an alkoxy radical and the other symbols are as hereinbefore defined.

Compounds of general formula X may also be prepared from compounds of general formula III wherein $R^{10}$ represents a cyano radical by methods known *per se* for the hydrolysis of a cyano radical to a carboxy radical, for example by treatment with concentrated hydrochloric acid. Compounds of general formula XI may be prepared by the reaction of a compound of general formula V (wherein the various symbols are as hereinbefore defined) with a compound of the general formula:—

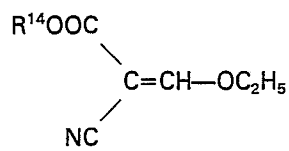

XII

wherein $R^{14}$ is as hereinbefore defined. Reaction may be effected at ambient temperature in acetic acid in the presence of sodium carbonate.

Compounds of general formulae V, VI and XII may be prepared by methods known *per se*.

Acid addition salts of compounds of general formula III wherein at least one of the symbols $R^5$, $R^6$, $R^7$, $R^8$ and $R^9$ represents a primary amino radical may be prepared from the corresponding compounds of general formula III by methods known *per se*, for example by reacting stoichiometric quantities of the compound of general formula III and the appropriate acid, for example an inorganic, e.g. hydrochloric acid, sulphuric acid, phosphoric acid or nitric acid, or an organic acid, e.g. acetic acid, in a suitable solvent. The acid addition salts may, if necessary, be purified by recrystallization from one, two or more suitable solvents.

As well as being useful in themselves as herbicidally active compounds, acid addition salts of aminophenyl compounds of general formula III may also be used in the purification of the corresponding compounds of general formula III, for example by exploitation of the solubility differences between the salts and the parent compounds in water and in organic solvents, by techniques which are well known to those skilled in the art.

By the term 'methods known *per se*', as used in the present specification is meant methods heretofore used or described in the chemical literature.

The N-phenylpyrazole derivatives of general formula III wherein $R^5$, $R^6$, $R^7$, $R^8$, $R^9$ and $R^{10}$ are as hereinbefore defined and, when at least one of $R^5$, $R^6$, $R^7$, $R^8$ and $R^9$ represents a primary amino group, acid addition salts thereof, with the exception of 5-amino-4-cyano-1-(2,4-dichlorophenyl)pyrazole, 5-amino-4-cyano-1-(4-chloro-2-methylphenyl)pyrazole, 5-amino-4-cyano-1-(2,4,5-trichlorophenyl)pyrazole, 5-amino-4-cyano-1-(2,4-dinitrophenyl)pyrazole and 5-amino-4-cyano-1-(2,4,6-trichlorophenyl)pyrazole, are new compounds which form a further feature of the present invention.

The following Examples and Reference Examples illustrate the preparation of compounds of general formula III.

Example 10

Pentafluorophenylhydrazine (10.3 g; described by Stacey *et al.*, J. Chem. Soc. *1960*, 1768) was added in portions to a refluxing solution of ethoxymethylenemalononitrile [6.35 g; described by Huber, J. Am. Chem. Soc. *65*, 2224 (1943)] in ethanol (23 ml). The solution was heated at reflux for one hour, cooled and diluted with water (23 ml) to precipitate a red solid which was crystallised firstly from aqueous methanol (50 ml, 1:1) and then toluene (25 ml). 5-Amino-4-cyano-1-pentafluorophenylpyrazole (9.8 g), m.p. 152—153°C, was obtained in the form of brown crystals.

By proceeding in a similar manner but replacing the pentafluorophenylhydrazine by the hereinafter identified appropriately substituted phenylhydrazine, there were prepared:—

5-Amino-4-cyano-1-(2,4,6-trifluorophenyl)pyrazole, m.p. 156—157°C (after crystallisation from a mixture of ethanol and n-hexane), from 2,4,6-trifluorophenylhydrazine;

5-Amino-4-cyano-1(2,3,4,6-tetrafluorophenyl)pyrazole, m.p. 176—177°C (after crystallisation from toluene), from 2,3,4,6-tetrafluorophenylhydrazine;

5-Amino-4-cyano-1-(2,4-dichlorophenyl)pyrazole, m.p. 136—137°C [described by Southwick & Dhawan, J. Heter. Chem., *12*, 1200 (1975)];

5-Amino-4-cyano-1-(2,3,4-trichlorophenyl)pyrazole, m.p. 159°C (after crystallisation from a mixture of toluene and light petroleum, b.p. 100—120°C), from 2,3,4-trichlorophenylhydrazine;

5-Amino-4-cyano-1-(2,4,5-trichlorophenyl)pyrazole, m.p. 181—183°C (after crystallisation from ethanol), from 2,4,5-trichlorophenylhydrazine [described by Chattaway *et al.*, J. Chem. Soc., *1931* 1925];

5-Amino-4-cyano-1-(2,4,6-trichlorophenyl)pyrazole, m.p. 213—214°C (after crystallisation from a mixture of acetone and toluene), from 2,4,6-trichlorophenylhydrazine [described by Chatterway and Irving, J. Chem. Soc., *1931*, 1740];

5-Amino-4-cyano-1-(2,4,6-tribromophenyl)pyrazole, m.p. 194—196°C (after crystallisation from ethanol), from 2,4,6-tribromophenylhydrazine [described by Neufeld, Ann. *248*, 96 (1888)];

5-Amino-4-cyano-1-(2,4-dichloro-5-isopropoxyphenyl)pyrazole, m.p. 198—199°C (after crystallisation from toluene), from 2,4-dichloro-5-isopropoxyphenylhydrazine [described in West German Patent Specification No. 1,795,773 (1976)].

Example 11

2,3,4-Trichlorophenylhydrazine (21.15 g) was added in portions to a refluxing solution of ethoxymethylenemalononitrile (12.2 g) in ethanol (100 ml). The solution obtained was then heated at reflux for one hour, cooled and evaporated to dryness. The residue thus obtained was triturated with diethyl ether (100 ml) to give 5-amino-4-cyano-1-(2,3,4-trichlorophenyl)pyrazole (12.6 g), m.p. 158—160°C, in the form of light brown crystals.

Example 12

2,3,4,5-Tetrachlorophenylhydrazine (12.3 g) was heated with ethoxymethylenemalononitrile 6.4 g) in dimethylformamide (25 ml) at reflux for 10 minutes in the presence of sodium carbonate (0.1 g). The hot solution was treated with charcoal and filtered and evaporated to dryness. The residue was triturated with diethyl ether and the ether solution was decanted and diluted with hexane to precipitate dark yellow crystals which were recrystallised from toluene to give 5-amino-4-cyano-1-(2,3,4,5-tetrachlorophenyl)pyrazole (5.3 g), m.p. 168—169°C, in the form of off-white crystals.

By proceeding in a similar manner but replacing the 2,3,4,5-tetrachlorophenylhydrazine by pentachlorophenylhydrazine [described by Suschitzky *et al.*, J. Chem. Soc., (c) 1971, 167] there was prepared 5-amino-4-cyano-1-pentachlorophenylpyrazole, m.p. 230—232°C (after crystallisation from ethanol).

Example 13

2,3,4-Trichlorophenylhydrazine (21.15 g) and ethoxymethylenemalononitrile (12.8 g) in ethoxyethanol (100 ml) were heated at reflux for one hour. The solution was then cooled and diluted with water. The sticky solid precipitate thus obtained was crystallized from toluene (48 ml) to give 5-amino-4-cyano-1-(2,3,4-trichlorophenyl)pyrazole (15 g), m.p. 154—156°C, in the form of off-white crystals.

Example 14

2,3,4-Trichlorophenylhydrazine (106 g) was added at laboratory temperature to a stirred solution of

anhydrous sodium acetate (20.5 g) in glacial acetic acid (250 ml). Ethoxymethylenemalononitrile (61 g) was then added with stirring to the suspension thus obtained. Partial solution occurred within 5 minutes, after which a fine precipitate was formed. The mixture was stirred for one hour and then filtered. The solid obtained was washed successively with water, aqueous sodium bicarbonate solution and water and dried to give 2,3,4-trichlorophenylhydrazinomethylenemalononitrile (118 g), m.p. 149—155°C, in the form of a yellow powder.

The 2,3,4-trichlorophenylhydrazinomethylenemalononitrile thus obtained was then heated at reflux for one hour in ethoxyethanol (300 ml). The hot solution was treated with decolourising charcoal, filtered and diluted with water (100 ml). The pale yellow crystals which formed were separated, dried and recrystallised from toluene (400 ml) to give 5-amino-4-cyano-1-(2,3,4-trichlorophenyl)pyrazole (100 g), m.p. 159—160°C, in the form of colourless crystals.

By proceeding in a similar manner, but replacing the 2,3,4-trichlorophenylhydrazine by the hereinafter indicated appropriately substituted phenylhydrazines, there were prepared:—

5-Amino-4-cyano-1-(2-nitro-4-trifluoromethylphenyl)pyrazole, m.p. 234—236°C, in the form of yellow crystals, from 2-nitro-4-trifluoromethylphenylhydrazine [described in West German Patent Specification No. 2,446,218 (1976)], *via* 2-nitro-4-trifluoromethylphenylhydrazinomethylenemalononitrile (isolated as a khaki-coloured powder, m.p. 156—158°C);

5-Amino-1-(2-bromo-3,4-dichlorophenyl)-4-cyanopyrazole, m.p. 163—165°C, in the form of a colourless powder, after crystallisation from toluene, from 2-bromo-3,4-dichlorophenylhydrazine, *via* 2-bromo-3,4-dichlorophenylhydrazinomethylenemalononitrile (isolated in the form of a cream-coloured powder, m.p. (153—155°C);

5-Amino-4-cyano-1-(3,4-dichloro-2-methylphenyl)pyrazole, m.p. 152—154°C, in the form of a colourless powder, after crystallisation from a mixture of toluene and n-hexane, from 3,4-dichloro-2-methylphenylhydrazine, *via* 3,4-dichloro-2-methylphenylhydrazinomethylenemalononitrile (isolated in the form of a fawn-coloured powder, m.p. 120—125°C);

5-Amino-1-(3-bromo-2,4-dichlorophenyl)-4-cyanopyrazole, m.p. 154—156°C, in the form of colourless crystals, after crystallisation from toluene, from 3-bromo-2,4-dichlorophenylhydrazine, *via* 3-bromo-2,4-dichlorophenylhydrazinomethylenemalonitrile (isolated in the form of a cream-coloured powder, m.p. 154—155°C);

5-Amino-4-cyano-1-(2,4-dichloro-3-methylphenyl)pyrazole, m.p. 159—161°C, in the form of colourless crystals, after crystallisation from toluene, from 2,4-dichloro-3-methylphenylhydrazine, *via* 2,4-dichloro-3-methylphenylhydrazinomethylenemalononitrile (isolated in the form of a buff-coloured powder, m.p. 132—134°C);

5-Amino-4-cyano-1-(3-cyano-2,4-dichlorophenyl)pyrazole, m.p. 230—232°C, in the form of colourless crystals, after crystallization from toluene, from 3-cyano-2,4-dichlorophenylhydrazine, *via* 3-cyano-2,4-dichlorophenylhydrazinomethylenemalononitrile (isolated as a buff-coloured powder, m.p. 158—161°C);

5-Amino-1-(4-bromo-2,3-dichlorophenyl)-4-cyanopyrazole, m.p. 154—155°C, in the form of pale yellow crystals, after crystallisation from toluene, from 4-bromo-2,3-dichlorophenylhydrazine, *via* 4-bromo-2,3-dichlorophenylhydrazinomethylenemalononitrile (isolated as a pale yellow powder, m.p. 148—151°C);

5-Amino-1-(4-bromo-2-chloro-3-methylphenyl)-4-cyanopyrazole, m.p. 174—176°C, in the form of off-white crystals, after crystallization from toluene, from 4-bromo-2-chloro-3-methylphenylhydrazine, *via* 4-bromo-2-chloro-3-methylphenylhydrazinomethylenemalononitrile (isolated in the form of a pale brown powder, m.p. 142—146°C);

5-Amino-1-(2-chloro-3-cyano-4-methylphenyl)-4-cyanopyrazole, m.p. 206—209°C, in the form of buff-coloured crystals, after crystallisation from ethanol, from 2-chloro-3-cyano-4-methylphenylhydrazine, *via* 2-chloro-3-cyano-4-methylphenylhydrazinomethylenemalononitrile (isolated in the form of a buff-coloured powder, m.p. 158—161°C);

5-Amino-1-(3-chloro-2,4-dimethylphenyl)-4-cyanopyrazole, m.p. 172—173°C in the form of pale brown crystals, from 3-chloro-2,4-dimethylphenylhydrazine, *via* 3-chloro-2,4-dimethylphenylhydrazinomethylenemalononitrile (isolated as an orange-coloured powder, m.p. 134—136°C);

5-Amino-1-(2-bromo-4-chloro-3-methylphenyl)-4-cyanopyrazole, m.p. 167—169°C in the form of colourless crystals, after crystallisation from toluene, from 2-bromo-4-chloro-3-methylphenylhydrazine, *via* 2-bromo-4-chloro-3-methylphenylhydrazinomethylenemalononitrile (isolated in the form of a pale brown powder, m.p. 146—147°C);

5-Amino-4-cyano-1-(3-chloro-2,4-difluorophenyl)pyrazole, m.p. 177°C, in the from of yellow crystals, after crystallisation from toluene, from 3-chloro-2,4-difluorophenylhydrazine, *via* 3-chloro-2,4-difluorophenylhydrazinomethylenemalononitrile (isolated in the form of a cream coloured powder, m.p. 175°C).

By proceeding in a similar fashion to that hereinbefore described in the present Example but replacing the 2,3,4-trichlorophenylhydrazine by the hereinafter identified appropriate substituted phenylhydrazine, the following N-phenylpyrazoles according to general formula III were prepared without isolation and cyclisation of an intermediate substituted phenylhydrazinomethylenemalononitrile:—

5-Amino-4-cyano-1-(2,4-dichloro-3-methoxyphenyl)pyrazole, m.p. 185—187°C, in the form of off-white crystals, after crystallisation from diethyl ether, from 2,4-dichloro-3-methoxyphenylhydrazine;

5-Amino-4-cyano-1-(2,3-dichloro-4-methylphenyl)pyrazole, m.p. 162—163°C, in the form of colourless

crystals, after crystallisation from a mixture of cyclohexane and ethyl acetate, from 2,3-dichloro-4-methylphenylhydrazine;

5-Amino-1-(2-chloro-3,4-dimethylphenyl)-4-cyanopyrazole, m.p. 179—181°C, in the form of off-white crystals, after crystallisation from toluene, from 2-chloro-3,4-dimethylphenylhydrazine;

5-Amino-1-(3-chloro-2,4-dibromophenyl)-4-cyanopyrazole, m.p. 171—172°C, in the form of off-white crystals, after crystallisation from toluene, from 3-chloro-2,4-dibromophenylhydrazine;

5-Amino-1-(4-chloro-2,3-dimethylphenyl)-4-cyanopyrazole, m.p. 167—170°C, in the form of a salmon-pink coloured solid, from 4-chloro-2,3-dimethylphenylhydrazine;

5-Amino-1-(4-chloro-3-cyano-2-methylphenyl)-4-cyanopyrazole, m.p. 221—224°C in the form of a buff-coloured powder, from 4-chloro-3-cyano-2-methylphenylhydrazine, and

5-Amino-4-cyano-1-(4-trifluoromethylphenyl)pyrazole, m.p. 155—156.5°C, in the form of a fawn coloured crystalline solid, after recrystallisation from toluene, from 4-trifluoromethylphenylhydrazine.

Example 15

Monomethylamine gas was passed for 15 minutes into a stirred solution of 5-amino-1-(2,3,4-trichlorophenyl)pyrazol-4-yl carbonyl chloride (2.66 g) in toluene (100 ml) at 0°C. The reaction mixture was then allowed to return to laboratory temperature and evaporated to dryness. The brown solid residue thus obtained was triturated with toluene (20 ml), diethyl ether (20 ml) and water (50 ml) to give 5-amino-4-N-methylcarbonamido-1-(2,3,4-trichlorophenyl)pyrazole (1.1 g), m.p. 224—226° C, in the form of a fawn coloured powder.

By proceeding in a similar fashion but replacing the monomethylamine by an excess of monoethylamine, there was prepared 5-amino-4-N-ethylcarbonamido-1-(2,3,4-trichlorophenyl)pyrazole, m.p. 241—242°C, in the form of colourless crystals, after crystallisation from toluene.

Reference Example 1

Phenylhydrazines used in the preparation of N-phenylpyrazole derivatives according to general formula III described in Examples 10 to 15 were prepared as follows;—

(a) 2,3,4,6-Tetrafluoroaniline (27.5 g) was diazotised with sodium nitrite (12.5 g) in a mixture of concentrated hydrochloric acid (580 ml) and glacial acetic acid (110 ml). The filtered diazonium salt solution was reduced by the addition of a solution of stannous chloride dihydrate (108 g) in concentrated sulphuric acid (108 ml) at 0—10°C. The resulting hydrazine hydrochloride solution was evaporated to dryness and the residue was dissolved in water and basified by the addition of aqueous ammonia solution (S.G. 0.880). The precipitated solid was filtered off and the filter pad was well washed with diethyl ether. The ethereal washings were combined with ether extracts of the filtrate, dried over sodium sulphate, filtered and evaporated to dryness. Trituration of the residue with hexane gave 2,3,4,6-tetrafluorophenylhydrazine (18.3 g), m.p. 74—76°C, in the form of off-white crystals.

By proceeding in a similar manner but replacing the 2,3,4,6-tetrafluoroaniline by 2,4,6-trifluoroaniline there was prepared 2,4,6-trifluorophenylhydrazine, m.p. 57—58°C (after crystallisation from aqueous methanol). By proceeding in a similar manner but replacing the 2,3,4,6-tetrafluoroaniline by 2,3,4-trichloroaniline there was prepared, after filtration of the insoluble hydrazine hydrochloride, 2,3,4-trichlorophenylhydrazine, m.p. 142—143°C. By proceeding in a similar manner but replacing the 2,3,4,6-trifluoroaniline by 4-trifluoromethylaniline, there was prepared, after filtration of the insoluble hydrazine hydrochloride, 4-trifluoromethylphenylhydrazine, m.p. 63—65°C, in the form of light brown crystals.

(b) 2,3,4,5-Tetrachloroaniline (23.1 g) was heated at 55—60°C in glacial acetic acid (125 ml) to which a solution of sodium nitrite (7.9 g) in concentrated sulphuric acid (60 ml) was added over 15 minutes. After the addition, heating at 60°C was maintained for 15 minutes and the solution was cooled to 0—5°C. A solution of stannous chloride dihydrate (87.5 g) in concentrated hydrochloric acid (75 ml) was added at less than 10°C and the voluminous precipitate was filtered off and washed with saturated aqueous sodium chloride solution. The solid was treated with a mixture of ice and aqueous ammonia (S.G. 0.880) to give a solid white mass which was removed by filtration and dried at 80°C. The white powder was extracted with chloroform in a Soxhlet apparatus to give, after evaporation, 2,3,4,5-tetrachlorophenylhydrazine (21 g), m.p. 172—176°C.

(c) 2,3,4-Trichloroaniline (100 g) was dissolved with stirring in glacial acetic acid (875 ml) at 55—60°C. A solution of sodium nitrite (39.5 g) in concentrated sulphuric acid (300 ml) was then added over 15 minutes at 55—60°C to the solution thus obtained. The viscous mixture obtained was cooled to 5—10°C and a solution of stannous chloride dihydrate (437 g) in concentrated hydrochloric acid (375 ml) was added at 5—10°C over 20 minutes. A fine, off-white solid precipitated. To aid filtration, the mixture was warmed to 60°C, allowed to cool to laboratory temperature and then filtered. The precipitate was washed on the filter with saturated aqueous sodium chloride solution (100 ml). The damp powder thus obtained was added to a stirred mixture of aqueous ammonia (1.3 litres; S.G. 0.880) and ice. The fine slurry which formed was filtered and the precipitate obtained was dried at 80°C, and boiled twice with chloroform (2 × 1.5 litres). The chloroform extracts were combined and evaporated to dryness to give 2,3,4-trichlorophenylhydrazine (86 g), m.p. 142—143°C, in the form of a colourless powder.

By proceeding in a similar manner, but replacing the 2,3,4-trichloroaniline by the appropriate substituted aniline, there were prepared:—

2-Bromo-3,4-dichlorophenylhydrazine, m.p. 143—146°C, in the form of off-white crystals, from 2-bromo-3,4-dichloroaniline;

3,4-Dichloro-2-methylphenylhydrazine, m.p. 105—106°C, in the form of a buff-coloured powder, from 3,4-dichloro-2-methylaniline (described by Sylvester and Wynne, J. Chem. Soc., *1936*, 694);

3-Bromo-2,4-dichlorophenylhydrazine, m.p. 128—130°C, in the form of a pink crystalline solid, from 3-bromo-2,4-dichloroaniline [described by Hurtley, J. Chem. Soc., *79*, 1302 (1901)];

2,4-Dichloro-3-methylphenylhydrazine, m.p. 58—60°C, in the form of yellow crystals, from 2,4-dichloro-3-methylaniline [described by Cohen and Dakin, J. Chem. Soc., *81*, 1332 (1902)];

2,4-Dichloro-3-methoxyphenylhydrazine, m.p. 88—90°C, in the form of brown crystals, from 2,4-dichloro-3-methoxyaniline;

3-Cyano-2,4-dichlorophenylhydrazine, m.p. 199—200°C, in the form of a yellow powder, from 3-cyano-2,4-dichloroaniline [described by N.V. Philips Gloelampenfabricken, Chem. Abs., *75*, P 5527K (1971)];

4-Bromo-2,3-dichlorophenylhydrazine, m.p. 135—137°C, in the form of a cream-coloured powder, from 4-bromo-2,3-dichloroaniline [described by Hurtley, J. Chem. Soc., *79*, 1302 (1901)];

2,3-Dichloro-4-methylphenylhydrazine, m.p. 111—114°C, in the form of colourless crystals, from 2,3-dichloro-4-methylaniline (described by Sylvester and Wynne, J. Chem. Soc., *1936*, 691);

4-Bromo-2-chloro-3-methylphenylhydrazine, m.p. 100—103°C, in the form of colourless solid, from 4-bromo-2-chloro-3-methylaniline;

2-Chloro-3,4-dimethylphenylhydrazine, m.p. 71—73°C, in the form of a buff-coloured powder, from 2-chloro-3,4-dimethylaniline (described by Hinkel *et al.*, J. Chem. Soc., *1934*, 286);

2-Chloro-3-cyano-4-methylphenylhydrazine, m.p. 168—170°C, in the form of a colourless powder, from 2-chloro-3-cyano-4-methylaniline;

3-Chloro-2,4-dibromophenylhydrazine, m.p. 148—150°C, in the form of brown crystals, after crystallisation from ethanol, from 3-chloro-2,4-dibromoaniline [described by Hurtley, J. Chem. Soc., *79*, 1304 (1901)];

3-Chloro-2,4-dimethylphenylhydrazine, m.p. 104°C, in the form of a yellow powder, from 3-chloro-2,4-dimethylaniline [described by Staskun, Tetrahedron, Vol. 28, 5069 (1972)];

2-Bromo-4-chloro-3-methylphenylhydrazine, m.p. 72—73°C, in the form of an off-white powder, from 2-bromo-4-chloro-3-methylaniline;

4-Chloro-2,3-dimethylphenylhydrazine, m.p. 69—71°C, in the form of a pale yellow powder, from 4-chloro-2,3-dimethylaniline (described by Hinkel *et al.,* J. Chem. Soc., *123*, 2968 (1923);

4-Chloro-3-cyano-2-methylphenylhydrazine, m.p. 184—187°C, in the form of colourless crystals, from 4-chloro-3-cyano-2-methylaniline, and

2,3,4,5-Tetrachlorophenylhydrazine, m.p. 172—176°C, in the form of a colourless powder, from 2,3,4,5-tetrachloroaniline.

Reference Example 2

Substituted anilines, not hitherto described in the chemical literature, used in the preparation of substituted phenylhydrazines as hereinbefore described in Reference Example 1 were prepared as follows:—

(a) A solution of 2-bromo-3,4-dichloronitrobenzene (23.5 g) in toluene was treated at 25—35°C for 1.25 hours with hydrogen in the presence of charcoal containing 5% platinum. The solution was then filtered and the filtrate was evaporated to dryness. The brown solid thus obtained was dissolved in diethyl ether and the solution was treated with decolourising charcoal and filtered. Evaporation to dryness of the filtrate gave 2-bromo-3,4-dichloroaniline (18.0 g), m.p. 78—80°C in the form of a brown powder.

By proceeding in a similar manner but replacing the 2-bromo-3,4-dichloronitrobenzene by the appropriately substituted nitrobenzene hereinafter indicated, there were prepared:—

2,4-Dichloro-3-methoxyaniline, in the form of a dark oil, from 2,4-dichloro-3-methoxynitrobenzene [described by Mallory and Varimbi, J. Org. Chem., *28*, 1656 (1963)];

4-Bromo-2-chloro-3-methylaniline, m.p. 51—52°C, in the form of light brown crystals, from 4-bromo-2-chloro-3-methylnitrobenzene, and

2-Bromo-4-chloro-3-methylaniline, m.p. 64—65°C, in the fom of an off-white solid, from 2-bromo-4-chloro-3-methylnitrobenzene.

(b) A solution of 2-chloro-6-methyl-3-nitrobenzonitrile (6.3 g) in ethanol (23 ml) was added to a solution of stannous chloride dihydrate (21.7 g) in concentrated hydrochloric acid (19 ml). The mixture was then heated at reflux for 15 minutes, cooled and diluted with water (200 ml). The fine white powder which precipitated was separated, dried and crystallized from ethanol (10 ml) to give 2-chloro-3-cyano-4-methylaniline (1.7 g), m.p. 117—119°C, in the form of colourless crystals.

By proceeding in a similar manner, but replacing the 2-chloro-6-methyl-3-nitrobenzonitrile by 6-chloro-2-methyl-3-nitrobenzonitrile, 4-chloro-3-cyano-2-methylaniline, m.p. 133—134°C, was obtained in the form of fluffy colourless crystals, after crystallisation from toluene.

Reference Example 3

Substituted nitrobenzenes, not hitherto described in the chemical literature, used in the preparation of substituted anilines as hereinbefore described in Reference Example 2, were prepared as follows:—

(a) 2,3-Dichloro-6-nitroaniline [29 g; prepared by a modification (reaction at 125°C) of the procedure described by Beilstein and Kurbatow, Ann., *192*, 235 (1878)] was dissolved with stirring at 55—60°C in glacial acetic acid (350 ml). A solution of sodium nitrite (11 g) in concentrated sulphuric acid (80 ml) was then added to the solution thus obtained with stirring over 15 minutes at a temperature of 55—60°C maintained by intermittent cooling with water. The red solution obtained was cooled to 15°C and poured, with stirring, into a solution of cuprous bromide (20 g) in concentrated hydrobromic acid (200 ml) and ice (500 g). The mixture was allowed to attain laboratory temperature, heated for one hour at 60°C and then steam-distilled. The distillate obtained was extracted with diethyl ether (6 × 500 ml). The combined ethereal extracts were washed successively with aqueus sodium hydroxide solution (2N; 3 × 750 ml) and water (2 × 500 ml), dried over anhydrous sodium sulphate and filtered. The filtrate was evaporated to dryness to give a yellow powder, which was then crystallised from hexane (125 ml) to give 2-bromo-3,4-dichloronitrobenzene (26.7 g), m.p. 73°C, in the form of yellow crystals.

By proceeding in a similar manner but replacing the 2,3-dichloro-6-nitroaniline by the appropriately substituted anilines hereafter indicated, there were prepared:—

4-Bromo-2-chloro-3-methylnitrobenzene, m.p. 59—60°C, in the form of yellow crystals, from 3-chloro-2-methyl-4-nitroaniline, and

2-Bromo-4-chloro-3-methylnitrobenzene, m.p. 43—44°C, in the form of yellow crystals, from 3-chloro-2-methyl-6-nitroalinine.

(b) 6-Chloro-2-methylbenzonitrile (29.7 g) was aded in small portions at −15 to −10°C, with stirring, to fuming nitric acid (150 ml; S.G. 1.52). The solution obtained was maintained at this temperature for 10 minutes, then allowed to stand at laboratory temperature for 24 hours and then poured on to ice (500 g). The yellow precipitate obtained was filtered off, washed with water and dried to give a mixture of nitrobenzonitriles (36.2 g), m.p. 76—80°C. The mixture of nitrobenzonitriles obtained (27.8 g) was chromatographed on a silica column (1 kg) eluted with a mixture of hexane and gradually increasing amounts (from 10% to 25% by volume) of ethyl acetate.

Evaporation of the eluate containing the faster-moving component gave 6-chloro-2-methyl-3-nitrobenzonitrile (20 g), m.p. 89—91°C, in the form of fluffy colourless crystals. Evaporation of the eluate containing the slower-moving component gave 2-chloro-6-methyl-3-nitrobenzonitrile (6 g), m.p. 106—107°C, in the form of a cream-coloured powder. (It is reported in British Patent Specification No. 1,141,249 (1969), that nitration of 6-chloro-2-methylbenzonitrile gives exclusively a product described as 6-chloro-2-methyl-3-nitrobenzonitrile, m.p. 75—80°C. However, the procedure described above leads unequivocally to the two products which were obtained).

Reference Example 4

The chloro-methyl-nitroanilines used in the preparation of bromo-chloro-methylnitrobenzenes as hereinbefore described in Reference Example 3 were prepared as follows:—

3-Chloro-*o*-toluidine (90 g) in a mixture of glacial acetic acid (90 ml) and acetic anhydride (90 ml) was heated on a steam bath for 10 minutes. The solution obtained was then poured into water. A colourless powder precipitated, which was filtered off and dried to give 3-chloro-2-methylacetanilide (104 g), m.p. 156—159°C, which was then dissolved with stirring in concentrated sulphuric acid (230 ml), while maintaining the temperature at not more than 30°C. The stirred solution was maintained at 5—10°C whilst concentrated nitric acid (38 ml; S.G. 1.42) was added during 30 minutes. The solution obtained was then stirred at 0°C for one hour and then poured on to ice. The solid precipitate which was obtained was separated by filtration, washed with water and dried, to give a mixture of nitro-acetanilides (145 g), m.p. 135—147°C, in the form of a yellow powder. This mixture of nitroacetanilides (94 g) was then heated at reflux for 6 hours in a mixture of glacial acetic acid (560 ml) and concentrated hydrochloric acid (370 ml). The mixture was then cooled, poured into water (1200 ml) and basified by the addition of aqueous 50% sodium hydroxide solution. The yellow precipitate which was obtained was separated by filtration, washed with water and dried, to give a mixture of nitroanilines (72 g), m.p. 150—164°C. This mixture was then steam-distilled in the presence of concentrated hydrochloric acid (200 ml). The solid which separated from the distillate was filtered off to give 3-chloro-2-methyl-4-nitroaniline (19.1 g), m.p. 152—154°C, in the form of a yellow solid. Further steam distillation gave a distillate containing a mixture of products (8.6 g), m.p. 134—138°C. The insoluble material, which had not been distilled, was separated from the acid solution to give 3-chloro-2-methyl-6-nitroaniline (18 g), m.p. 164—165°C, in the form of a brown powder.

Reference Example 5

5-Amino-1-(2,3,4-trichlorophenyl)pyrazol-4-yl carbonyl chloride used in the preparation of N-phenylpyrazole derivatives according to general formula III described in Example 15 was prepared as follows:—

5-Amino-1-(2,3,4-trichlorophenyl)pyrazol-4-yl carboxylic acid (54 g) and thionyl chloride (75 ml) were heated together under reflux for one hour. The reaction mixture was then evaporated to dryness and residual thionyl chloride was removed by evaporation in the presence of toluene (3 × 200 ml) to give 5-amino-1-(2,3,4-trichlorophenyl)pyrazol-4-yl carbonyl chloride, in the form of a dark coloured oil which was used in the preparation described in Example 11 without further purification.

5-Amino-1-(2,3,4-trichlorophenyl)pyrazol-4-yl carboxylic acid used in the above preparation was prepared as follows:—

2,3,4-Trichlorophenylhydrazine (53 g) and ethyl 2-cyano-3-ethoxyprop-2-enoate [45.6 g; described by De Bollemont, Comte Rendu, *128*, 1339 (1889)] were added to a stirred solution of sodium acetate (10 g) in acetic acid (150 ml) at laboratory temperature. After stirring for 30 minutes, the reaction mixture was diluted with diethyl ether (500 ml) and the solid material was removed by filtration. The separated solid was suspended in diethyl ether (500 ml) and filtered to give ethyl 2-cyano-3-(2,3,4-trichlorophenylhydrazo)prop-2-enoate (70 g), m.p. 177—178°C, in the form of a colourless crystalline solid.

Ethyl 2-cyano-3-(2,3,4-trichlorophenylhydrazo)prop-2-enoate (70 g) was heated under reflux in ethoxyethanol (375 ml) for 1.5 hours. The hot solution was then filtered and the filtrate was diluted with water (1.5 litres). The solid which precipitated was removed by filtration to give ethyl 2-amino-1-(2,3,4-trichlorophenyl)pyrazol-4-ylcarboxylate (65 g), m.p. 158—159°C, in the form of a colourless crystalline solid.

Ethyl 2-amino-1-(2,3,4-trichlorophenyl)pyrazol-4-ylcarboxylate prepared as described above (73 g) was heated under reflux in a solution of potassium hydroxide (120 g) in water (1 litre) for 4 hours with vigorous stirring. The orange coloured solution thus obtained was then filtered. The filtrate was washed with diethyl ether (2 × 500 ml) and acidified to pH 1 by the addition of concentrated hydrochloric acid. The solid which precipitated was separated by filtration, washed with water and dried, to give 5-amino-1-(2,3,4-trichlorophenyl)pyrazol-4-yl carboxylic acid (52 g), m.p. 186°C (with decomposition), in the form of a cream coloured powder.

2,3,4,6-Tetrafluoroaniline, 2,3,4-trichloroaniline and 2,3,4,5-tetrachloroaniline, used in the preparation of substituted phenylhydrazines as hereinbefore described in Reference Example 1, and 6-chloro-2-methylbenzonitrile, used in the preparation of chloro-methyl-nitrobenzonitriles as hereinbefore described in Reference Example 3, are known compounds which are readily available.

**Claims for the Contracting States: BE CH DE FR GB IT LI LU NL SE**

1. A herbicidal composition characterised in that it contains, as active ingredient, at least one N-phenylpyrazole derivative of the general formula:—

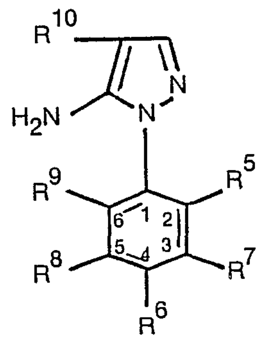

III

(wherein each of the symbols $R^5$ and $R^6$, which may be the same or different, represents an alkyl or alkoxy radical containing from 1 to 4 carbon atoms, a trifluoromethyl, trifluoromethoxy, nitro, cyano or primary amino radical, or a fluorine, chlorine or bromine atom, each of the symbols $R^7$, $R^8$ and $R^9$, which may be the same or different, represents a hydrogen atom, an alkyl or alkoxy radical containing from 1 to 4 carbon atoms, a trifluoromethyl, trifluoromethoxy, nitro, cyano or primary amino radical or a fluorine, chlorine or bromine atom, or the symbols $R^5$, $R^7$, $R^8$ and $R^9$ each represents a hydrogen atom and the symbol $R^6$ represents a trifluoromethoxy or trifluoromethyl radical, and the symbol $R^{10}$ represents a cyano radical or substituted carbamoyl radical —$CONHR^{11}$, wherein $R^{11}$ represents a methyl or ethyl radical), or when at least one of symbols $R^5$, $R^6$, $R^7$, $R^8$ and $R^9$ represents a primary amino radical an agriculturally acceptable acid addition salt thereof, with the exclusion of 5-amino-4-cyano-1-(2,4,6-trichlorophenyl)pyrazole, in association with one or more compatible herbicidally-acceptable diluents or carriers, with the proviso that when the N-phenylpyrazole of general formula III is 5 - amino - 4 - cyano - 1 - (2,4 - dichlorophenyl)-pyrazole, 5 - amino - 4 - cyano - 1 - (2,4,5 - trichlorophenyl)pyrazole, 5 - amino - 4 - cyano - 1 - (4 - chloro - 2 - methylphenyl)pyrazole or 5 - amino - 4 - cyano - 1 - (2,4 - dinitrophenyl)pyrazole, the herbicidal composition is not an association of the N-phenylpyrazole derivative solely with water or a common organic solvent.

2. A herbicidal composition according to claim 1 characterised in that the active ingredient is an N-phenylpyrazole derivative of the general formula depicted in claim 1 wherein each of the symbols $R^5$ and $R^6$, which may be the same or different, represents a methyl, trifluoromethyl or nitro radical or a fluorine, chlorine, or bromine atom, $R^7$ represents a methyl, methoxy or cyano radical or a fluorine, chlorine or bromine atom, $R^8$ and $R^9$ represent hydrogen atoms and $R^{10}$ represents a cyano radical.

3. A herbicidal composition according to claim 1 characterised in that the active ingredient is 5-amino-4-cyano-1-(2,3,4-trichlorophenyl)pyrazole.

4. A herbicidal composition according to claim 1, 2 or 3 characterised in that it contains from 0.05 to

90% by weight of N-phenylpyrazole derivative(s).

5. A herbicidal composition according to claim 1 characterised in that it is in the form of an aqueous suspension concentrate comprising from 10 to 70% w/v of at least one N-phenylpyrazole derivative of the general formula depicted in claim 1, from 2 to 10% w/v of surface-active agent, from 0.1 to 5% w/v of thickener and from 15 to 87.9% by volume of water.

6. A herbicidal composition according to claim 1 characterised in that it is in the form of a wettable powder comprising from 10 to 90% w/w of at least one N-phenylpyrazole derivative of the general formula depicted in claim 1, from 2 to 10% w/w of surface-active agent, and from 10 to 88% w/w of solid diluent or carrier.

7. A herbicidal composition according to claim 1 characterised in that it is in the form of a liquid water soluble concentrate comprising from 10 to 30% w/v of at least one N-phenylpyrazole derivative of the general formula depicted in claim 1, from 5 to 25% w/v of surface-active agent and from 45 to 85% by volume of water-miscible solvent.

8. A herbicidal composition according to claim 1 characterised in that it is in the form of a liquid emulsifiable suspension concentrate comprising from 10 to 70% w/v of at least one N-phenylpyrazole derivative of the general formula depicted in claim 1, from 5 to 15% w/v of surface-active agent, from 0.1 to 5% w/v of thickener and from 10 to 84.9% by volume of organic solvent.

9. A herbicidal composition according to claim 1 characterised in that it is in the form of granules comprising from 2 to 10% w/w of at least one N-phenylpyrazole derivative of the general formula depicted in claim 1, from 0.5 to 2% w/w of surface-active agent and from 88 to 97.5% w/w of granular carrier.

10. A method of controlling the growth of weeds at a locus characterised in that a herbicidal composition as claimed in claim 1, 2, 3 or 4 is applied to the locus at a rate sufficient to give between 0.1 kg and 20 kg of N-phenylpyrazole derivative per hectare.

11. An N-phenylpyrazole derivative characterised in that it is of the general formula depicted in claim 1 (wherein the symbols $R^5$, $R^6$, $R^7$, $R^8$, $R^9$ and $R^{10}$ are as defined in claim 1) and, when at least one of symbols $R^5$, $R^6$, $R^7$, $R^8$ and $R^9$ represents a primary amino radical, agriculturally acceptable acid addition salts thereof, with the exclusion of 5 - amino - 4 - cyano - 1 - (2,4 - dichlorophenyl)pyrazole, 5 - amino - 4 - cyano - 1 - (2,4,5 - trichlorophenyl)pyrazole, 5 - amino - 4 - cyano - 1 - (4 - chloro - 2 - methylphenyl)-pyrazole, 5 - amino - 4 - cyano - 1 - (2,4 - dinitrophenyl)pyrazole and 5 - amino - 4 - cyano - 1 - (2,4,6 - trichlorophenyl)pyrazole.

12. An N-phenylpyrazole derivative according to claim 11 characterised in that it is of the general formula depicted in claim 1 wherein each of the symbols $R^5$ and $R^6$, which may be the same or different, represents a methyl, trifluoromethyl or nitro radical or a fluorine, chlorine or bromine atom $R^7$ represents a methyl, methoxy or cyano radical or a fluorine, chlorine or bromine atom, $R^8$ and $R^9$ represent hydrogen atoms and $R^{10}$ represents a cyano radical.

13. An N-phenylpyrazole derivative according to claim 11 characterised in that it is 5-amino-4-cyano-1-(2,3,4-trichlorophenyl)pyrazole.

14. A process for the preparation of an N-phenylpyrazole derivative as claimed in claim 11, wherein $R^5$, $R^6$, $R^7$, $R^8$ and $R^9$ are as defined in claim 1 and $R^{10}$ represents a cyano radical, with the exclusion of 5 - amino - 4 - cyano - 1 - (2,4 - dichlorophenyl)pyrazole, 5 - amino - 4 - cyano - 1 - (2,4,5 - trichlorophenyl)pyrazole, 5 - amino - 4 - cyano - 1 - (4 - chloro - 2 - methylphenyl)pyrazole, 5 - amino - 4 - cyano - 1 - (2,4 - dinitrophenyl)pyrazole and 5 - amino - 4 - cyano - 1 - (2,4,6 - trichlorophenyl)pyrazole, characterised in that it comprises:

(A) the cyclisation of a compound of the general formula:—

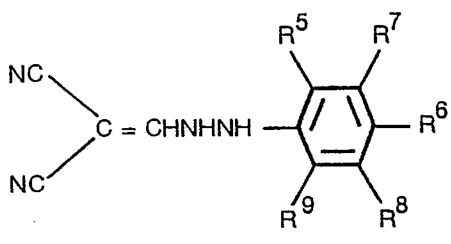

IV

wherein the various symbols are as defined in claim 1 with the provisos that (i) $R^5$ and $R^6$ are other than chlorine atoms when $R^7$, $R^8$ and $R^9$ are hydrogen atoms, (ii) $R^5$ is other than the methyl radical when $R^6$ is a chlorine atom and $R^7$, $R^8$ and $R^9$ are hydrogen atoms, (iii) $R^5$ is other than a chlorine atom when $R^6$ and $R^8$ each represents a chlorine atom and $R^7$ and $R^9$ each represent a hydrogen atom, (iv) $R^5$ is other than a nitro radical when $R^6$ is a nitro radical and $R^7$, $R^8$ and $R^9$ each represent a hydrogen atom and (v) $R^5$ is other than a chlorine atom when $R^6$ and $R^9$ each represent a chlorine atom and $R^7$ and $R^8$ each represent a hydrogen atom, or

(B) the reaction of a compound of the general formula:—

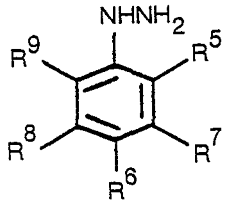

V

(wherein the various symbols are as defined in claim 1 with the provisos that (i) $R^5$ and $R^6$ are other than chlorine atoms when $R^7$, $R^8$ and $R^9$ are hydrogen atoms, (ii) $R^5$ is other than the methyl radical when $R^6$ is a chlorine atom and $R^7$, $R^8$ and $R^9$ are hydrogen atoms, (iii) $R^5$ is other than a chlorine atom when $R^6$ and $R^8$ each represent a chlorine atom and $R^7$ and $R^9$ each represent a hydrogen atom, (iv) $R^5$ is other than a nitro radical when $R^6$ is a nitro radical and $R^7$, $R^8$ and $R^9$ each represent a hydrogen atom and (v) $R^5$ is other than a chlorine atom when $R^6$ and $R^9$ each represent a chlorine atom and $R^7$ and $R^8$ each represent a hydrogen atom), or an acid addition salt thereof, with a compound of the general formula:—

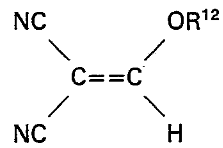

(VI)

wherein $R^{12}$ represents a straight- or branched-chain alkyl radical containing from 1 to 4 carbon atoms, optionally followed by the step of conversion by methods known *per se* of a so obtained N-phenylpyrazole derivative of the general formula depicted in claim 1, wherein at least one of the symbols $R^5$, $R^6$, $R^7$, $R^8$ and $R^9$ represents a primary amino radical, into an acid addition salt.

15. A process for the preparation of an N-phenylpyrazole derivative as claimed in claim 11, wherein $R^5$, $R^6$, $R^7$, $R^8$ and $R^9$ are as defined in claim 1 except for the primary amino and cyano radicals and $R^{10}$ represents a substituted carbamoyl group —$CONHR^{11}$ (wherein $R^{11}$ is as defined in claim 1), characterised in that it comprises the selective alkylation of a compound of the general formula:—

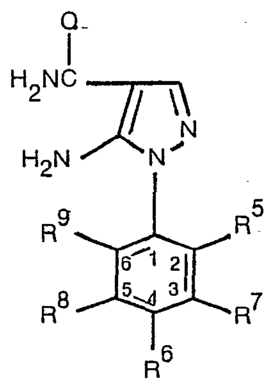

VII

(wherein $R^5$, $R^6$, $R^7$, $R^8$ and $R^9$ are as defined in claim 1 except for the primary amino and cyano radicals) with methyl iodide or ethyl iodide, in the presence of an inorganic base.

16. A process for the preparation of an N-phenylpyrazole derivative as claimed in claim 11, wherein $R^5$, $R^6$, $R^7$, $R^8$ and $R^9$ are as defined in claim 1 and $R^{10}$ represents a substituted carbamoyl group —$CONHR^{11}$ (wherein $R^{11}$ is as defined in claim 1), characterised in that it comprises the reaction of a compound of the general formula:—

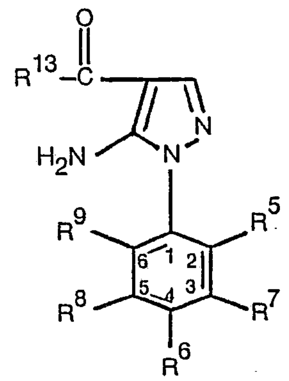

VIII

(wherein $R^5$, $R^6$, $R^7$, $R^8$ and $R^9$ are as defined in claim 1, and $R^{13}$ represents a chlorine atom or a straight- or branched-chain alkoxy radical containing from 1 to 4 carbon atoms) with an amine of the general formula:—

$$H_2NR^{11} \qquad IX$$

wherein $R^{11}$ represents a methyl or ethyl radical, optionally followed by the step of conversion by methods known *per se* of an N-phenylpyrazole derivative of the general formula depicted in claim 1, wherein at least one of the symbols $R^5$, $R^6$, $R^7$, $R^8$ and $R^9$ represents a primary amino radical, so obtained into an acid addition salt.

17. Compounds of general formula IV depicted in claim 14 wherein the various symbols are as defined in claim 1 with the provisos that (i) $R^5$ and $R^6$ are other than chlorine atoms when $R^7$, $R^8$ and $R^9$ are hydrogen atoms, (ii) $R^5$ is other than the methyl radical when $R^6$ is a chlorine atom and $R^7$, $R^8$ and $R^9$ are hydrogen atoms, (iii) $R^5$ is other than a chlorine atom when $R^6$ and $R^8$ each represent a chlorine atom and $R^7$ and $R^9$ each represent a hydrogen atom, (iv) $R^5$ is other than a nitro radical when $R^6$ is a nitro radical and $R^7$, $R^8$ and $R^9$ each represent a hydrogen atom and (v) $R^5$ is other than a chlorine atom when $R^6$ and $R^9$ each represent a chlorine atom and $R^7$ and $R^8$ each represent a hydrogen atom.

**Claims for the Contracting State: AT**

1. A herbicidal composition characterised in that it contains, as active ingredient, at least one N-phenylpyrazole derivative of the general formula:—

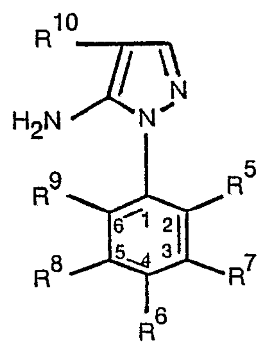

III

(wherein each of the symbols $R^5$ and $R^6$, which may be the same or different, represents an alkyl or alkoxy radical containing from 1 to 4 carbon atoms, a trifluoromethyl, trifluoromethoxy, nitro, cyano or primary amino radical, or a fluorine, chlorine or bromine atom, each of the symbols $R^7$, $R^8$ and $R^9$, which may be the same or different, represents a hydrogen atom, an alkyl or alkoxy radical containing from 1 to 4 carbon atoms, a trifluoromethyl, trifluoromethoxy, nitro, cyano or primary amino radical or a fluorine, chlorine or bromine atom, or the symbols $R^5$, $R^7$, $R^8$ and $R^9$ each represents a hydrogen atom and the symbol $R^6$ represents a trifluoromethoxy or trifluoromethyl radical, and the symbol $R^{10}$ represents a cyano radical or substituted carbamoyl radical —$CONHR^{11}$, wherein $R^{11}$ represents a methyl or ethyl radical), or when at least one of symbols $R^5$, $R^6$, $R^7$, $R^8$ and $R^9$ represents a primary amino radical an agriculturally acceptable acid addition salt thereof, with the exclusion of 5-amino-4-cyano-1-(2,4,6-trichlorophenyl)pyrazole, in association with one or more compatible herbicidally-acceptable diluents or carriers, with the proviso that when the N-phenylpyrazole of general formula III is 5 - amino - 4 - cyano - 1 - (2,4 - dichlorophenyl)-pyrazole, 5 - amino - 4 - cyano - 1 - (2,4,5 - trichlorophenyl)pyrazole, 5 - amino - 4 - cyano - 1 - (4 - chloro - 2 - methylphenyl)pyrazole or 5 - amino - 4 - cyano - 1 - (2,4 - dinitrophenyl)pyrazole, the

herbicidal composition is not an association of the N-phenylpyrazole derivative solely with water or a common organic solvent.

2. A herbicidal composition according to claim 1 characterised in that the active ingredient is an N-phenylpyrazole derivative of the general formula depicted in claim 1 wherein each of the symbols $R^5$ and $R^6$, which may be the same or different, represents a methyl, trifluoromethyl or nitro radical or a fluorine, chlorine, or bromine atom, $R^7$ represents a methyl, methoxy or cyano radical or a fluorine, chlorine or bromine atom, $R^8$ and $R^9$ represent hydrogen atoms and $R^{10}$ represents a cyano radical.

3. A herbicidal composition according to claim 1 characterised in that the active ingredient is 5-amino-4-cyano-1-(2,3,4-trichlorophenyl)pyrazole.

4. A herbicidal composition according to claim 1, 2 or 3 characterised in that it contains from 0.05 to 90% by weight of N-phenylpyrazole derivative(s).

5. A herbicidal composition according to claim 1 characterised in that it is in the form of an aqueous suspension concentrate comprising from 10 to 70% w/v of at least one N-phenylpyrazole derivative of the general formula depicted in claim 1, from 2 to 10% w/v of surface-active agent, from 0.1 to 5% w/v of thickener and from 15 to 87.9% by volume of water.

6. A herbicidal composition according to claim 1 characterised in that it is in the form of a wettable powder comprising from 10 to 90% w/w of at least one N-phenylpyrazole derivative of the general formula depicted in claim 1, from 2 to 10% w/w of surface-active agent, and from 10 to 88% w/w of solid diluent or carrier.

7. A herbicidal composition according to claim 1 characterised in that it is in the form of a liquid water soluble concentrate comprising from 10 to 30% w/v of at least one N-phenylpyrazole derivative of the general formula depicted in claim 1, from 5 to 25% w/v of surface-active agent and from 45 to 85% by volume of water-miscible solvent.

8. A herbicidal composition according to claim 1 characterised in that it is in the form of a liquid emulsifiable suspension concentrate comprising from 10 to 70% w/v of at least one N-phenylpyrazole derivative of the general formula depicted in claim 1, from 5 to 15% w/v of surface-active agent, from 0.1 to 5% w/v of thickener and from 10 to 84.9% by volume of organic solvent.

9. A herbicidal composition according to claim 1 characterised in that it is in the form of granules comprising from 2 to 10% w/w of at least one N-phenylpyrazole derivative of the general formula depicted in claim 1, from 0.5 to 2% w/w of surface-active agent and from 88 to 97.5% w/w of granular carrier.

10. A method of controlling the growth of weeds at a locus characterised in that a herbicidal composition as claimed in claim 1, 2, 3 or 4 is applied to the locus at a rate sufficient to give between 0.1 kg and 20 kg of N-phenylpyrazole derivative per hectare.

11. A process for the preparation of an N-phenylpyrazole derivative of the general formula depicted in claim 1, wherein $R^5$, $R^6$, $R^7$, $R^8$ and $R^9$ are as defined in claim 1 and $R^{10}$ represents a cyano radical, with the exclusion of 5 - amino - 4 - cyano - 1 - (2,4 - dichlorophenyl)pyrazole, 5 - amino - 4 - cyano - 1 - (2,4,5 - trichlorophenyl)pyrazole, 5 - amino - 4 - cyano - 1 - (4 - chloro - 2 - methylphenyl)pyrazole, 5 - amino - 4 - cyano - 1 - (2,4 - dinitrophenyl)pyrazole and 5 - amino - 4 - cyano - 1 - (2,4,6 - trichlorophenyl)pyrazole, characterised in that it comprises:

(A) the cyclisation of a compound of the general formula:—

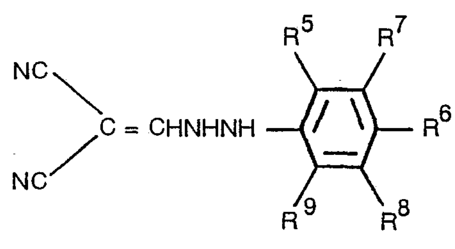

IV

wherein the various symbols are as defined in claim 1 with the provisos that (i) $R^5$ and $R^6$ are other than chlorine atoms when $R^7$, $R^8$ and $R^9$ are hydrogen atoms, (ii) $R^5$ is other than the methyl radical when $R^6$ is a chlorine atom and $R^7$, $R^8$ and $R^9$ are hydrogen atoms, (iii) $R^5$ is other than a chlorine atom when $R^6$ and $R^8$ each represents a chlorine atom and $R^7$ and $R^9$ each represent a hydrogen atom, (iv) $R^5$ is other than a nitro radical when $R^6$ is a nitro radical and $R^7$, $R^8$ and $R^9$ each represent a hydrogen atom and (v) $R^5$ is other than a chlorine atom when $R^6$ and $R^9$ each represent a chlorine atom and $R^7$ and $R^8$ each represent a hydrogen atom, or (B) the reaction of a compound of the general formula:—

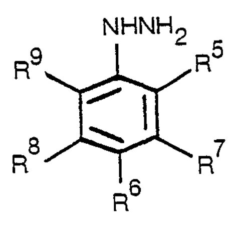

V

(wherein the various symbols are as defined in claim 1 with the provisos that (i) $R^5$ and $R^6$ are other than chlorine atoms when $R^7$, $R^8$ and $R^9$ are hydrogen atoms, (ii) $R^5$ is other than the methyl radical when $R^6$ is a chlorine atom and $R^7$, $R^8$ and $R^9$ are hydrogen atoms, (iii) $R^5$ is other than a chlorine atom when $R^6$ and $R^8$ each represent a chlorine atom and $R^7$ and $R^9$ each represent a hydrogen atom, (iv) $R^5$ is other than a nitro radical when $R^6$ is a nitro radical and $R^7$, $R^8$ and $R^9$ each represent a hydrogen atom and (v) $R^5$ is other than a chlorine atom when $R^6$ and $R^9$ each represent a chlorine atom and $R^7$ and $R^8$ each represent a hydrogen atom), or an acid addition salt thereof, with a compound of the general formula:—

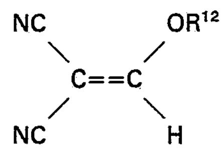

(VI)

wherein $R^{12}$ represents a straight- or branched-chain alkyl radical containing from 1 to 4 carbon atoms, optionally followed by the step of conversion by methods known *per se* of a so obtained N-phenylpyrazole derivative of the general formula depicted in claim 1, wherein at least one of the symbols $R^5$, $R^6$, $R^7$, $R^8$ and $R^9$ represents a primary amino radical, into an acid addition salt.

12. A process for the preparation of an N-phenylpyrazole derivative of the general formula depicted in claim 1, wherein $R^5$, $R^6$, $R^7$, $R^8$ and $R^9$ are as defined in claim 1 except for the primary amino and cyano radicals and $R^{10}$ represents a substituted carbamoyl group —$CONHR^{11}$ (wherein $R^{11}$ is as defined in claim 1), characterised in that it comprises the selective alkylation of a compound of the general formula:—

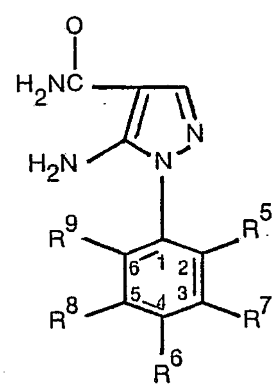

VII

(wherein $R^5$, $R^6$, $R^7$, $R^8$ and $R^9$ are as defined in claim 1 except for the primary amino and cyano radicals) with methyl iodide or ethyl iodide, in the presence of an inorganic base.

13. A process for the preparation of an N-phenylpyrazole derivative of the general formula depicted in claim 1, wherein $R^5$, $R^6$, $R^7$, $R^8$ and $R^9$ are as defined in claim 1 and $R^{10}$ represents a substituted carbamoyl group —$CONHR^{11}$ (wherein $R^{11}$ is as defined in claim 1), characterised in that it comprises the reaction of a compound of the general formula:—

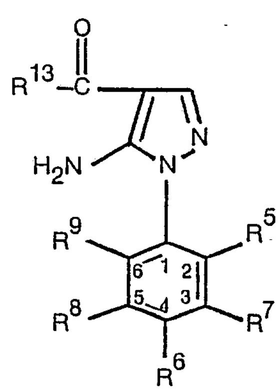

VIII

(wherein $R^5$, $R^6$, $R^7$, $R^8$ and $R^9$ are as defined in claim 1, and $R^{13}$ represents a chlorine atom or a straight- or branched-chain alkoxy radical containing from 1 to 4 carbon atoms) with an amine of the general formula:—

$$H_2NR^{11} \quad \text{IX}$$

wherein $R^{11}$ represents a methyl or ethyl radical, optionally followed by the step of conversion by methods known *per se* of an N-phenylpyrazole derivative of the general formula depicted in claim 1, wherein at least one of the symbols $R^5$, $R^6$, $R^7$, $R^8$ and $R^9$ represents a primary amino radical, so obtained into an acid addition salt.

**Patentansprüche für die Vertragsstaaten: BE CH DE FR GB IT LI LU NL SE**

1. Herbizide Mittel, dadurch gekennzeichnet, dass sie als Wirkstoff mindestens ein N-Phenylpyrazolderivat der allgemeinen Formel

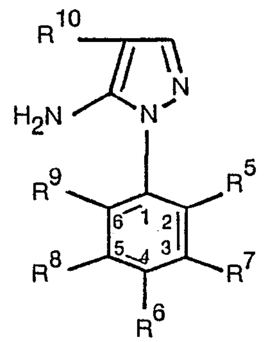

III

(worin die Symbole $R^5$ und $R^6$, die gleich oder verschieden sein können, jeweils eine Alkyl- oder Alkoxygruppe mit 1 bis 4 Kohlenstoffatomen, eine Trifluormethyl-, Trifluormethoxy-, Nitro-, Cyano- oder primäre Aminogruppe oder ein Fluor-, Chlor- oder Bromatom darstellen, wobei die Symbole $R^7$, $R^8$ und $R^9$, die gleich oder verschieden sein können, jeweils ein Wasserstoffatom, eine Alkyl- oder Alkoxygruppe mit 1 bis 4 Kohlenstoffatomen, eine Trifluormethyl-, Trifluormethoxy-, Nitro-, Cyano- oder primäre Aminogruppe oder ein Fluor-, Chlor- oder Bromatom darstellen, oder die Symbole $R^5$, $R^7$, $R^8$ und $R^9$ je ein Wasserstoffatom und das Symbol $R^6$ eine Trifluormethoxy- oder Trifluormethylgruppe darstellen, und das Symbol $R^{10}$ eine Cyanogruppe oder eine substituierte Carbamoylgruppe —$CONHR^{11}$ darstellt, worin $R^{11}$ eine Methyl- oder Aethylgruppe darstellt), oder, falls wenigstens eines der Symbole $R^5$, $R^6$, $R^7$, $R^8$ und $R^9$ eine primäre Amino-gruppe darstellt, deren landwirtschaftlich annehmbare Säureadditionssalze, unter Ausschluss von 5-Amino-4-cyano-1-(2,4,6-trichlorphenyl)pyrazol, in Verbindung mit einem oder mehreren verträglichen, für Herbizide unbedenklichen Verdünnungsmitteln oder Trägerstoffen enthalten, mit der Massgabe, dass, wenn das N-Phenylpyrazol der allgemeinen Formel III 5 - Amino - 4 - cyano - 1 - (2,4 - dichlorphenyl)-pyrazol, 5 - Amino - 4 - cyano - 1 - (2,4,5 - trichlorphenyl)pyrazol, 5 - Amino - 4 - cyano - 1 - (4 - chlor - 2 - methylphenyl)pyrazole oder 5 - Amino - 4 - cyano - 1 - (2,4 - dinitrophenyl)pyrazol ist, das herbizide Mittel kein Gemenge des N-Phenylpyrazolderivats nur mit Wasser oder einem üblichen organischen Lösungsmittel ist.

2. Herbizide Mittel nach Anspruch 1, dadurch gekennzeichnet, dass der Wirkstoff ein N-Phenylpyrazolderivat der in Anspruch 1 gezeigten allgemeinen Formel ist, in der die Symbole $R^5$ und $R^6$, die gleich oder verschieden sein können, jeweils eine Methyl-, Trifluormethyl- oder Nitrogruppe oder ein Fluor-, Chlor- oder Bromatom darstellen, $R^7$ eine Methyl-, Methoxy- oder Cyanogruppe oder ein Fluor-, Chlor- oder Bromatom darstellt, $R^8$ und $R^9$ Wasserstoffatome und $R^{10}$ eine Cyanogruppe darstellen.

3. Herbizide Mittel nach Anspruch 1, dadurch gekennzeichnet, dass der Wirkstoff 5-Amino-4-cyano-1-(2,3,4-trichlorphenyl)pyrazol ist.

4. Herbizide Mittel nach Anspruch 1, 2 oder 3, dadurch gekennzeichnet, dass sie 0,05 bis 90 Gew.-% des (der) N-Phenylpyrazolderivats (-derivate) enthalten.

5. Herbizide Mittel nach Anspruch 1, dadurch gekennzeichnet, dass sie in Form eines wässrigen Suspensionskonzentrats vorliegen, das 10 bis 70 Gew./Vol.-% mindestens eines N-Phenylpyrazolderivats der in Anspruch 1 gezeigten allgemeinen Formel, 2 bis 10 Gew./Vol.-% eines oberflächenaktiven Mittels, 0,1 bis 5 Gew./Vol.-% eines Verdickungsmittels und 15 bis 87,9 Vol.-% Wasser enthält.

6. Herbizide Mittel nach Anspruch 1, dadurch gekennzeichnet, dass sie in Form eines Spritzpulvers vorliegen, das 10 bis 90 Gew./Gew.-% mindestens eines N-Phenylpyrazolderivats der in Anspruch 1 gezeigten allgemeinen Formel, 2 bis 10 Gew./Gew.-% eines oberflächenaktiven Mittels und 10 bis 88 Gew./Gew.-% eines festen Verdünnungsmittels oder Trägerstoffs enthält.

7. Herbizide Mittel nach Anspruch 1, dadurch gekennzeichnet, dass sie in Form eines flüssigen wasserlöslichen Konzentrats vorliegen, das 10 bis 30 Gew./Vol-% mindestens eines N-Phenylpyrazolderivats der in Anspruch 1 gezeigten allgemeinen Formel, 5 bis 25 Gew./Vol.-% eines oberflächenaktiven Mittels und 45 bis 85 Vol.-% eines mit Wasser mischbaren Lösungsmittels enthält.

8. Herbizide Mittel nach Anspruch 1, dadurch gekennzeichnet, dass sie in Form eines flüssigen emulgierbaren Suspensionskonzentrats vorliegen, das 10 bis 70 Gew./Vol.-% mindestens eines N-Phenylpyrazolderivats der in Anspruch 1 gezeigten allgemeinen Formel, 5 bis 15 Gew./Vol.-% eines oberflächenaktiven Mittels, 0,1 bis 5 Gew./Vol.-% eines Verdickungsmittels und 10 bis 84,9 Vol.-% eines organischen Lösungsmittels enthält.

9. Herbizide Mittel nach Anspruch 1, dadurch gekennzeichnet, dass sie in Form eines Granulats vorliegen, das 2 bis 10 Gew./Gew.-% mindestens eines N-Phenylpyrazolderivats der in Anspruch 1 gezeigten allgemeinen Formel, 0,5 bis 2 Gew./Gew.-% eines oberflächenaktiven Mittels und 88 bis 97,5 Gew./Gew.-% eines granulatförmigen Trägerstoffs enthält.

10. Verfahren zur Beherrschung des Unkrautwachstums an einem Ort, dadurch gekennzeichnet, dass man an dem Ort ein herbizides Mittel nach Anspruch 1, 2, 3 oder 4 in einer ausreichenden Menge von 0,1 bis 20 kg N-Phenylpyrazolderivat pro Hektar aufbringt.

11. N-Phenylpyrazolderivat, dadurch gekennzeichnet, dass es die in Anspruch 1 gezeigte allgemeine Formel aufweist (worin die Symbole $R^5$, $R^6$, $R^7$, $R^8$, $R^9$ und $R^{10}$ wie in Anspruch 1 definiert sind) und, wenn wenigstens eines der Symbole $R^5$, $R^6$, $R^7$, $R^8$ und $R^9$ eine primäre Aminogruppe darstellt, deren landwirtschaftlich annehmbare Säureadditionssalze, unter Ausschluss von 5 - Amino - 4 - cyano - 1 - (2,4 - dichlorphenyl)pyrazol, 5 - Amino - 4 - cyano - 1 - (2,4,5 - trichlorphenyl)pyrazol, 5 - Amino - 4 - cyano - 1 - (4 - chlor - 2 - methylphenyl)pyrazol, 5 - Amino - 4 - cyano - 1 - (2,4 - dinitrophenyl)pyrazol und 5 - Amino - 4 - cyano - 1 - (2,4,6 - trichlorpphenyl)pyrazol.

12. N-Phenylpyrazolderivat nach Anspruch 11, dadurch gekennzeichnet, dass es die in Anspruch 1 gezeigte allgemeine Formel aufweist, worin jedes der Symbole $R^5$ und $R^6$, die gleich oder verschieden sein können, eine Methyl-, Trifluormethyl- oder Nitrogruppe oder ein Fluor-, Chlor- oder Bromatom darstellt, $R^7$ einen Methyl-, Methoxy- oder Cyanorest oder ein Fluor-, Chlor- oder Bromatom darstellt, $R^8$ und $R^9$ Wasserstoffatome und $R^{10}$ eine Cyanogruppe darstellen.

13. N-Phenylpyrazolidérivat nach Anspruch 11, dadurch gekennzeichnet, dass es 5-Amino-4-cyano-1-(2,3,4-trichlorphenyl)pyrazol ist.

14. Verfahren zur Herstellung eines N-Phenylpyrazolderivats nach Anspruch 11, worin $R^5$, $R^6$, $R^7$, $R^8$ und $R^9$ wie in Anspruch 1 definiert sind und $R^{10}$ eine Cyanogruppe darstellt, unter Ausschluss von 5 - Amino - 4 - cyano - 1 - (2,4 - dichlorphenyl)pyrazol, 5 - Amino - 4 - cyano - 1 - (2,4,5 - trichlorphenyl)pyrazol, 5 - Amino - 4 - cyano - 1 - (4 - chlor - 2 - methylphenyl)pyrazol, 5 - Amino - 4 - cyano - 1 - (2,4 - dinitrophenyl)pyrazol und 5 - Amino - 4 - cyano - 1 - (2,4,6 - trichlorphenyl)pyrazol, gekennzeichnet durch die folgenden Schritte:

A) Ringschluss einer Verbindung der allgemeinen Formel

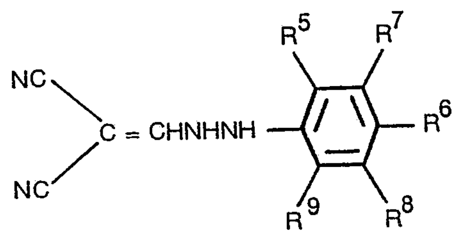

IV

worin die verschiedenen Symbole wie in Anspruch 1 definiert sind, mit den Massgaben, dass (i) $R^5$ und $R^6$ keine Chloratome sind, wenn $R^7$, $R^8$ und $R^9$ Wasserstoffatome darstellen, (ii) $R^5$ keine Methylgruppe ist, wenn $R^6$ ein Chloratom und $R^7$, $R^8$ und $R^9$ Wasserstoffatome darstellen, (iii) $R^5$ kein Chloratom ist, wenn $R^6$ und $R^8$ jeweils ein Chloratom und $R^7$ und $R^9$ jeweils ein Wasserstoffatom darstellen, (iv) $R^5$ keine Nitrogruppe ist, wenn $R^6$ eine Nitrogruppe ist und $R^7$, $R^8$ und $R^9$ jeweils ein Wasserstoffatom darstellen, und (v) $R^5$ kein Chloratom ist, wenn $R^6$ und $R^9$ jeweils ein Chloratom und $R^7$ und $R^8$ jeweils ein Wasserstoffatom darstellen, oder

B) Umsetzung einer Verbindung der allgemeinen Formel

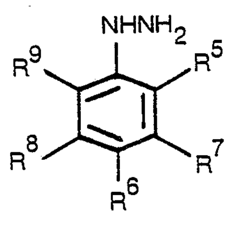

V

(worin die verschiedenen Symbole wie in Anspruch 1 definiert sind, mit den Massgaben, dass (i) $R^5$ und $R^6$ keine Chloratome sind, wenn $R^7$, $R^8$ und $R^9$ Wasserstoffatome darstellen, (ii) $R^5$ keine Methylgruppe ist, wenn $R^6$ ein Chloratom und $R^7$, $R^8$ und $R^9$ Wasserstoffatome darstellen, (iii) $R^5$ kein Chloratom ist, wenn $R^6$ und $R^8$ jeweils ein Chloratom und $R^7$ und $R^9$ jeweils ein Wasserstoffatom darstellen, (iv) $R^5$ keine Nitrogruppe ist, wenn $R^6$ eine Nitrogruppe ist und $R^7$, $R^8$ und $R^9$ jeweils ein Wasserstoffatom darstellen, und (v) $R^5$ kein Chloratom ist, wenn $R^6$ und $R^9$ jeweils ein Chloratom und $R^7$ und $R^8$ jeweils ein Wasserstoffatom darstellen);

oder deren Säureadditionssalze, mit einer Verbindung der allgemeinen Formel

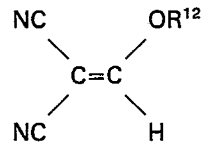

(VI)

in der $R^{12}$ eine verzweigte oder unverzweigte Alkylgruppe mit 1 bis 4 Kohlenstoffatomen darstellt, gegebenenfalls mit einer nachfolgenden Umwandlungsstufe, durch an sich bekannte Methoden, eines so gewonnenen N-Phenylpyrazolderivats der in Anspruch 1 gezeigten allgemeinen Formel, in der wenigstens eines der Symbole $R^5$, $R^6$, $R^7$, $R^8$ und $R^9$ eine primäre Aminogruppe darstellt, in ein Säureadditionssalz.

15. Verfahren zur Herstellung eines N-Phenylpyrazolderivats nach Anspruch 11 worin $R^5$, $R^6$, $R^7$, $R^8$, und $R^9$ mit Ausnahme der primären Amino- und Cyanogruppen die in Anspruch 1 genannte Bedeutung haben und $R^{10}$ eine substituierte Carbamoylgruppe —CONHR$^{11}$ darstellt (in der $R^{11}$ die in Anspruch 1 genannte Bedeutung besitzt), dadurch gekennzeichnet, dass eine Verbindung der allgemeinen Formel

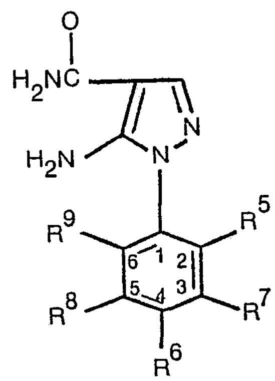

VII

(in der $R^5$, $R^6$, $R^7$, $R^8$ und $R^9$ mit Ausnahme der primären Amino- und Cyanogruppen die in Anspruch 1 genannte Bedeutung besitzen) mit Methyljodid oder Aethyljodid in Gegenwart einer anorganischen Base selektiv alkyliert wird.

16. Verfahren zur Herstellung eines N-Phenylpyrazolderivats nach Anspruch 11, worin $R^5$, $R^6$, $R^7$, $R^8$ und $R^9$ wie in Anspruch 1, definiert sind und $R^{10}$ eine substituierte Carbamoylgruppe —CONHR$^{11}$ darstellt (in der $R^{11}$ die in Anspruch 1 genannte Bedeutung besitzt), dadurch gekennzeichnet, dass eine Verbindung der allgemeinen Formel

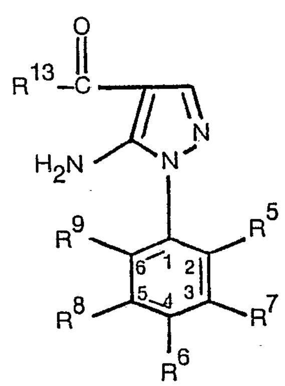

VIII

(in der $R^5$, $R^6$, $R^7$, $R^8$ und $R^9$ die in Anspruch 1 genannte Bedeutung besitzen und $R^{13}$ ein Chloratom oder eine unverzweigte oder verzweigte Alkoxygruppe mit 1 bis 4 Kohlenstoffatomen darstellen), mit einem Amin der allgemeinen Formel

$$H_2NR^{11} \qquad IX$$

in der $R^{11}$ eine Methyl- oder Aethylgruppe darstellt, umgesetzt wird, gegebenenfalls gefolgt von einer Umwandlungsstufe, durch an sich bekannte Methoden, eines so gewonnenen N-Phenylpyrazolderivats der in Anspruch 1 gezeigten allgemeinen Formel, worin wenigstens eines der Symbole $R^5$, $R^6$, $R^7$, $R^8$ und $R^9$

eine primäre Aminogruppe darstellt, in ein Säureadditionssalz.

17. Verbindungen der in Anspruch 14 gezwigten allgemeinen Formel IV, worin die verschiedenen Symbole wie in Anspruch 1 definiert sind, mit den Massgaben, dass (i) $R^5$ und $R^6$ keine Chloratome sind, wenn $R^7$, $R^8$ und $R^9$ Wasserstoffatome darstellen, (ii) $R^5$ keine Methylgruppe ist, wenn $R^6$ ein Chloratom und $R^7$, $R^8$ und $R^9$ Wasserstoffatome darstellen, (iii) $R^5$ kein Chloratom ist, wenn $R^6$ und $R^8$ jeweils ein Chloratom und $R^7$ und $R^9$ jeweils ein Wasserstoffatom darstellen, (iv) $R^5$ keine Nitrogruppe ist, wenn $R^6$ eine Nitrogruppe ist und $R^7$, $R^8$ und $R^9$ jeweils ein Wasserstoffatom darstellen, und (v) $R^5$ kein Chloratom ist, wenn $R^6$ und $R^9$ jeweils ein Chloratom und $R^7$ und $R^8$ jeweils ein Wasserstoffatom darstellen.

**Patentansprüche für den Vertragsstaat: AT**

1. Herbizide Mittel, dadurch gekennzeichnet, dass sie als Wirkstoff mindestens ein N-Phenylpyrazolderivat der allgemeinen Formel

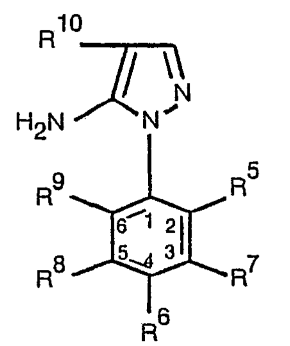

III

(worin die Symbole $R^5$ und $R^6$, die gleich oder verschieden sein können, jeweils eine Alkyl- oder Alkoxygruppe mit 1 bis 4 Kohlenstoffatomen, eine Trifluormethyl-, Trifluormethoxy-, Nitro-, Cyano- oder primäre Aminogruppe oder ein Fluor-, Chlor- oder Bromatom darstellen, wobei die Symbole $R^7$, $R^8$ und $R^9$, die gleich oder verschieden sein können, jeweils ein Wasserstoffatom, eine Alkyl- oder Alkoxygruppe mit 1 bis 4 Kohlenstoffatomen, eine Trifluormethyl-, Trifluormethoxy-, Nitro-, Cyano- oder primäre Aminogruppe oder ein Fluor-, Chlor- oder Chromatom darstellen, oder die Symbole $R^5$, $R^7$, $R^8$ und $R^9$ je ein Wasserstoffatom und das Symbol $R^6$ eine Trifluormethoxy- oder Trifluormethylgruppe darstellen, und das Symbol $R^{10}$ eine Cyanogruppe oder eine substituierte Carbamoylgruppe —$CONHR^{11}$ darstellen, worin $R^{11}$ eine Methyl- oder Aethylgruppe darstellt, oder, falls wenigstens eines der Symbole $R^5$, $R^6$, $R^7$, $R^8$ und $R^9$ eine primäre Amino-gruppe darstellt, deren landwirtschaftlich annehmbare Säureadditionssalze, unter Ausschluss von 5-Amino-4-cyano-1-(2,4,6-trichlorphenyl)pyrazol, in Verbindung mit einem oder mehreren verträglichen, für Herbizide unbedenklichen Verdünnungsmitteln oder Trägerstoffen enthalten, mit der Massgabe, dass, wenn das N-Phenylpyrazol der allgemeinen Formel III 5 - Amino - 4 - cyano - 1 - (2,4 - dichlorphenyl)-pyrazol, 5 - Amino - 4 - cyano - 1 - (2,4,5) - trichlorphenyl)pyrazol, 5 - Amino - 4 - cyano - 1 - (4 - chlor - 2 - methylphenyl)pyrazol oder 5 - Amino - 4 - cyano - 1 - (2,4 - dinitrophenyl)pyrazol ist, das herbizide Mittel kein Gemenge des N-Phenylpyrazolderivats nur mit Wasser oder einem üblichen organischen Lösungsmittel ist.

2. Herbizide Mittel nach Anspruch 1, dadurch gekennzeichnet, dass der Wirkstoff ein N-Phenylpyrazolderivat der in Anspruch 1 gezeigten allgemeinen Formel ist, in der die Symbole $R^5$ und $R^6$, die gleich oder verschieden sein können, jeweils eine Methyl-, Trifluormethyl- oder Nitrogruppe oder ein Fluor-, Chlor- oder Bromatom darstellen, $R^7$ eine Methyl-, Methoxy- oder Cyanogruppe oder ein Fluor-, Chlor- oder Bromatom darstellen, $R^8$ und $R^9$ Wasserstoffatome und $R^{10}$ eine Cyanogruppe darstellen.

3. Herbizide Mittel nach Anspruch 1, dadurch gekennzeichnet, dass der Wirkstoff 5-Amino-4-cyano-1-(2,3,4-trichlorphenyl)pyrazol ist.

4. Herbizide Mittel nach Anspruch 1, 2 oder 3, dadurch gekennzeichnet, dass sie 0,05 bis 90 Gew.-% des (der) N-Phenylpyrazolderivats (-derivate) enthalten.

5. Herbizide Mittel nach Anspruch 1, dadurch gekennzeichnet, dass sie in Form eines wässrigen Suspensionskonzentrats vorliegen, das 10 bis 70 Gew./Vol.-% mindestens eines N-Phenylpyrazolderivats der in Anspruch 1 gezeigten allgemeinen Formel, 2 bis 10 Gew./Vol.-% eines oberflächenaktiven Mittels, 0,1 bis 5 Gew./Vol.-% eines Verdickungsmittels und 15 bis 87,9 Vol.-% Wasser enthält.

6. Herbizide Mittel nach Anspruch 1, dadurch gekennzeichnet, dass sie in Form eines Spritzpulvers vorliegen, das 10 bis 90 Gew./Gew.-% mindestens eines N-Phenylpyrazolderivats der in Anspruch 1 gezeigten allgemeinen Formel, 2 bis 10 Gew./Gew.-% eines oberflächenaktiven Mittels und 10 bis 88 Gew./Gew.-% eines festen Verdünnungsmittels oder Trägerstoffs enthält.

7. Herbizide Mittel nach Anspruch 1, dadurch gekennzeichnet, dass sie in Form eines flüssigen wasserlöslichen Konzentrats vorliegen, das 10 bis 30 Gew./Vol-% mindestens eines N-Phenylpyrazolderivats der in Anspruch 1 gezeigten allgemeinen formel, 5 bis 25 Gew./Vol.-% eines oberflächenaktiven Mittels und 45 bis 85 Vol.-% eines mit Wasser mischbaren Lösungsmittels enthält.

8. Herbizide Mittel nach Anspruch 1, dadurch gekennzeichnet, dass sie in Form eines flüssigen emulgierbaren Suspensionskonzentrats vorliegen, das 10 bis 70 Gew.-/Vol.-% mindestens eines N-Phenylpyrazolderivats der in Anspruch 1 gezeigten allgemeinen Formel, 5 bis 15 Gew./Vol.-% eines oberflächenaktiven Mittels, 0,1 bis 5 Gew./Vol.-% eines Verdickungsmittels und 10 bis 84,9 Vol.-% eines organischen Lösungsmittels enthält.

9. Herbizide Mittel nach Anspruch 1, dadurch gekennzeichnet, dass sie in Form eines Granulats vorliegen, das 2 bis 10 Gew./Gew.-% mindestens N-Phenylpyrazolderivats der in Anspruch 1 gezeigten allgemeinen Formel, 0,5 bis 2 Gew./Gew.-% eines oberflächenaktiven Mittels und 88 bis 97,5 Gew./Gew.-% eines granulatförmigen Trägerstoffs enthält.

10. Verfahren zur Beherrschung des Unkrautwachstums an einem Ort, dadurch gekennzeichnet, dass man an dem Ort ein herbizides Mittel nach Anspruch 1, 2, 3 oder 4 in einer ausreichenden Menge von 0,1 bis 20 kg N-Phenylpyrazolderivat pro Hektar aufbringt.

11. Verfahren zur Herstellung eines N-Phenylpyrazolderivats der in Anspruch 1 gezeigten allgemeinen Formel, worin $R^5$, $R^6$, $R^7$, $R^8$ und $R^9$ wie in Anspruch 1 definiert sind und $R^{10}$ eine Cyanogruppe darstellt, unter Ausschluss von 5 - Amino - 4 - cyano - 1 - (2,4, - dichlorphenyl)pyrazol, 5 - Amino - 4 - cyano - 1 - (2,4,5 - trichlorphenyl)pyrazol, 5 - Amino - 4 - cyano -1 - (4 - chlor - 2 - methylphenyl)pyrazol, 5 - Amino - 4 - cyano - 1 - (2,4 - dinitrophenyl)pyrazol und 5 - Amino - 4 - cyano - 1 - (2,4,6 - trichlorphenyl)pyrazol, gekennzeichnet durch die folgenden Schritte:

A) Ringschluss einer Verbindung der allgemeinen Formel

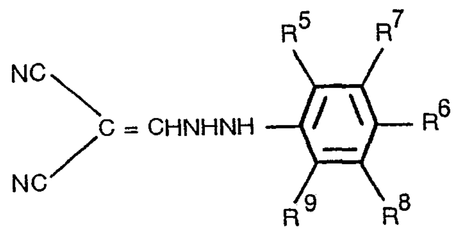

IV

worin die verschiedenen Symbole wie in Anspruch 1 definiert sind, mit den Massgaben, dass (i) $R^5$ und $R^6$ keine Chloratome sind, wenn $R^7$, $R^8$ und $R^9$ Wasserstoffatome darstellen, (ii) $R^5$ keine Methylgruppe ist, wenn $R^6$ ein Chloratom und $R^7$, $R^8$ und $R^9$ Wasserstoffatome darstellen, (iii) $R^5$ kein Chloratom ist, wenn $R^6$ und $R^8$ jeweils ein Chloratom und $R^7$ und $R^9$ jeweils ein Wasserstoffatom darstellen, (iv) $R^5$ keine Nitrogruppe ist, wenn $R^6$ eine Nitrogruppe ist und $R^7$, $R^8$ und $R^9$ jeweils ein Wasserstoffatom darstellt, und (v) $R^5$ kein Chloratom ist, wenn $R^6$ und $R^9$ jeweils ein Chloratom und $R^7$ und $R^8$ jeweils ein Wasserstoffatom darstellen,

B) Umsetzung einer Verbindung der allgemeinen Formel

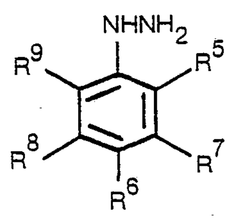

V

(worin die verschiedenen Symbole wie in Anspruch 1 definiert sind, mit den Massgaben, dass (i) $R^5$ und $R^6$ keine Chloratome sind, wenn $R^7$, $R^8$ und $R^9$ Wasserstoffatome darstellen, (ii) $R^5$ keine Methylgruppe ist, wenn $R^6$ ein Chloratom und $R^7$, $R^8$ und $R^9$ Wasserstoffatome darstellen, (iii) $R^5$ kein Chloratom ist, wenn $R^6$ und $R^8$ jeweils ein Chloratom und $R^7$ und $R^9$ jeweils ein Wasserstoffatom darstellen, (iv) $R^5$ keine Nitrogruppe ist, wenn $R^6$ eine Nitrogruppe ist und $R^7$, $R^8$ und $R^9$ jeweils ein Wasserstoffatom darstellen, und (v) $R^5$ kein Chloratom ist, wenn $R^6$ und $R^9$ jeweils ein Chloratom und $R^7$ und $R^8$ jeweils ein Wasserstoffatomen darstellen);

oder deren Säureadditionssalze, mit einer Verbindung der allgemeinen Formel

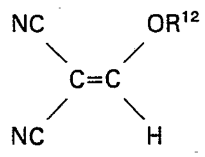

(VI)

in der $R^{12}$ eine verzeigte oder unverzweigte Alkylgruppe mit 1 bis 4 Kohlenstoffatomen darstellt, gegebenenfalls mit einer nachfolgenden Umwandlungsstufe, durch an sich bekannte Methoden, eines so

gewonnenen N-Phenylpyrazolderivats der in Anspruch 1 gezeigten allgemeinen Formel, in der wenigstens eines der Symbole $R^5$, $R^6$, $R^7$, $R^8$ und $R^9$ eine primäre Aminogruppe darstellt, in ein Säureadditionssalz.

12. Verfahren zur Herstellung eines N-Phenylpyrazolderivats nach Anspruch 1 gezeigten allgemeinen Formel, worin $R^5$, $R^6$, $R^7$, $R^8$, und $R^9$ mit Ausnahme der primären Amino- und Cyanogruppen die in Anspruch 1 genannte Bedeutung haben und $R^{10}$ eine substituierte Carbamoylgruppe —$CONHR^{11}$ darstellt (in der $R^{11}$ die in Anspruch 1 genannte Bedeutung besitz), dadurch gekennzeichnet, dass eine Verbindung der allgemeinen Formel

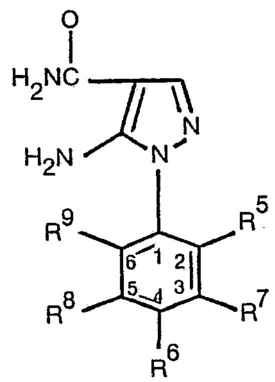

VII

(in der $R^5$, $R^6$, $R^7$, $R^8$, und $R^9$ mit Ausnahme der primären Amino- und Cyanogruppen die in Anspruch 1 genannte Bedeutung besitzen) mit Methyljodid oder Aethyljodid in Gegenwart einer anorganischen Base selektiv alkyliert wird.

13. Verfahren zur Herstellung eines N-Phenylpyrazolderivats nach Anspruch 11, worin $R^5$, $R^6$, $R^7$, $R^8$ und $R^9$ wie in Anspruch 1 definiert sind und $R^{10}$ eine substituierte Carbamoylgruppe —$CONHR^{11}$ darstellt (in der $R^{11}$ die in Anspruch 1 genannte Bedeutung besitzt), dadurch gekennzeichnet, dass eine Verbindung der allgemeinen Formel

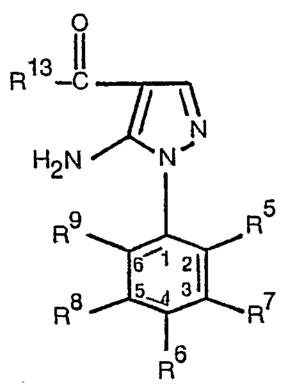

VIII

(in der $R^5$, $R^6$, $R^7$, $R^8$ und $R^9$ die in Anspruch 1 genannte Bedeutung besitzen und $R^{13}$ ein Chloratom oder eine unverzweigte oder verzweigte Alkoxygruppe mit 1 bis 4 Kohlenstoffatomen darstellen), mit einem Amin der allgemeinen Formel

$$H_2NR^{11} \qquad IX$$

in der $R^{11}$ eine Methyl- oder Aethylgruppe darstellt, umgesetzt wird, gegebenenfalls gefolgt von einer Umwandlungsstufe, durch an sich bekannte Methoden, eines so gewonnenen N-Phenylpyrazolderivats der in Anspruch 1 gezeigten allgemeinen Formel, worin wenigstens eines der Symbole $R^5$, $R^6$, $R^7$, $R^8$ und $R^9$ eine primäre Aminogruppe darstellt, in ein Säureadditionssalz.

**Revendications pour les Etats contractants: BE CH DE FR GB IT LI LU NL SE**

1. Composition herbicide caractérisée en ce qu'elle contient comme matière active, au moins un dérivé du N-phénylpyrazole de formule générale III:

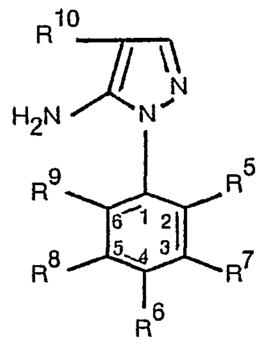

III

(dans laquelle:

— $R^5$ et $R^6$, identiques ou différents, représentent chacun un radical alcoyle ou alcoxy contenant de 1 à 4 atomes de carbone, un radical trifluorométhyle, trifluorométoxy, nitro, cyano ou amino primaire, ou un atome de fluor, chlore ou brome,

— $R^7$, $R^8$, et $R^9$, identiques ou différents, représentent chacun un atome d'hydrogène, un radical alcoyle ou alcoxy contenant de 1 à 4 atomes de carbone, un radical trifluorométhyle, trifluorométhoxy, nitro, cyano ou amino primaire, ou un atome de fluor, chlore ou brome,

— $R^5$, $R^7$, $R^8$ et $R^9$ pouvant de plus représenter chacun un atome d'hydrogène et $R^6$ le radical trifluorométhoxy ou trifluorométhyle,

— $R^{10}$ représente un radical cyano ou un radical carbamoyle substitué

$$-CONHR^{11}$$

dans lequel $R^{11}$ représente un radical méthyle ou éthyle), ou, lorsque au moins un des symboles $R^5$, $R^6$, $R^7$, $R^8$ et $R^9$ représente un radical amino primaire, un sel d'acide acceptable en agriculture, à l'exclusion du 5-amino-4-cyano-1-(2,4,6-trichlorophényl)pyrazole, en association avec un ou plus diluant ou support compatible et acceptable pour des herbicides, sous réserve que, quand le N-phénylepyrazole de formule générale III est le 5-amino-4-cyano-1-(2,4-dichlorophényl)pyrazole le 5-amino-4-cyano-1-(2,4,5-trichlorophényl)pyrazole, le 5-amino-4-cyano-1-(4-chloro-2-méthylphényl)pyrazole ou le 5-amino-4-cyano-1-(2,4-dinitrophényl)pyrazole, la composition herbicide n'est pas une association du dérivé N-phénylpyrazole seulement avec l'eau ou un solvant organique commun.

2. Composition herbicide selon la revendication 1 caractérisée en ce que la matière active est un dérivé du N-phénylpyrazole ayant la formule générale décrite à la revendication 1 dans laquelle:

— $R^5$ et $R^6$, identiques ou différents, représentent chacun un radical méthyle, trifluorométhyle, ou nitro ou un atome de fluor, chlore ou brome,

— $R^7$ représente un radical méthyle, méthoxy ou cyano ou un atome de fluor, chlore ou brome,

— $R^8$ et $R^9$ représentent chacun un atome d'hydrogène,

— $R^{10}$ représente un radical cyano.

3. Composition herbicide selon la revendication 1, caractérisée en ce que la matière active est le 5-amino-4-cyano-1-(2,3,4-trichlorophényl)pyrazole.

4. Composition herbicide selon l'une des revendications 1, 2 ou 3, caractérisée en ce qu'elle contient de 0,05 à 90% en poids du dérivé(s) du N-phénylpyrazole.

5. Composition herbicide selon la revendication 1, caractérisée en ce qu'elle se présente sous forme d'une suspension aqueuse concentrée contenant:

— de 10 à 70% en poids/volume d'au moins un dérivé du N-phénylpyrazole ayant la formule générale décrite à la revendication 1,

— de 2 à 10% en poids/volume d'un agent tensio-actif,

— de 0,1 à 5% en poids/volume d'un épaississant et

— de 15 à 87,9% en volume d'eau.

6. Composition herbicide selon la revendication 1, caractérisé en ce qu'elle se présente sous forme d'une poudre mouillable contenant:

— de 10 à 90% en poids d'au moins un dérivé du N-phénylpyrazole ayant la formule générale décrite à la revendication 1,

— de 2 à 10% en poids d'agent tensio-actif, et

— de 10 à 88% en poids d'un diluant ou un support solide.

7. Composition herbicide selon la revendication 1, caractérisée en ce qu'elle se présente sous forme d'un concentré liquide soluble dans l'eau contenant:

— de 10 à 30% en poids/volume d'au moins un dérivé du N-phénylpyrazole ayant la formule générale décrite à la revendication 1,

— de 5 à 25% en poids/volume d'agent tensio-actif, et

— de 45 à 85% en volume de solvant miscible à l'eau.

8. Composition herbicide selon la revendication 1, caractérisée en ce qu'elle se présente sous forme d'une suspension concentrée liquide émulsionnable contenant:

— de 10 à 70% en poids/volume d'au moins un dérivé du N-phénylpyrazole ayant la formule générale décrite à la revendication 1,

— de 5 à 15% en poids/volume d'agent tensio-actif,

— de 0,1 à 5% en poids/volume d'épaississant et

— de 10 à 84,9% en volume de solvant organique.

9. Composition herbicide selon la revendication 1, caractérisée en ce qu'elle se présente sous forme de granulés contenant:

— de 2 à 10% en poids/volume d'au moins un dérivé du N-phénylpyrazole ayant la formule générale décrite à la revendication 1,

— de 0,5 à 2% en poids d'agent tensio-actif,

— et de 88 à 97,5% en poids de support granulé.

10. Procédé de contrôle de la croissance des adventices en un lieu donné, caractérisé en ce qu'on applique en ce lieu, une composition herbicide selon l'une des revendications 1, 2, 3 et 4, à raison de 0,1 à 20 kg/ha de dérivé N-phénylpyrazole.

11. Dérivé du N-phénylpyrazole caractérisé en ce qu'il a la formule générale décrite à la revendication 1, (dans laquelle les symboles $R^5$, $R^6$, $R^7$, $R^8$, $R^9$ et $R^{10}$ sont comme définis à la revendication 1) et, quand au moins un des symboles $R^5$, $R^6$, $R^7$, $R^8$ et $R^9$ représente un radical amino primaire, les sels d'acide acceptables en agriculture, à l'exclusion des 5 - amino - 4 - cyano - 1 - (2,4 - dichlorophényl)pyrazole, 5 - amino - 4 - cyano - 1 - (2,4,5 - trichlorophényl)pyrazole, 5 - amino - 4 - cyano - 1 - (4 - chloro - 2 - méthylphényl)pyrazole, 5 - amino - 4 - cyano - 1 - (2,4 - dinitrophényl)pyrazole, et 5 - amino - 4 - cyano - 1 - (2,4,6 - trichlorophényl)pyrazole.

12. Dérivé du N-phénylpyrazole selon la revendication 11, caractérisé en ce qu'il a la formule générale décrite à la revendication 1, dans laquelle $R^5$ et $R^6$, identiques ou différents, représentent chacun un radical méthyle, trifluorométhyle ou nitro ou un atome de fluor, chlore ou brome, $R^7$ représente un radical méthyle, méthoxy, ou cyano ou un un atome de fluor, chlore ou brome, $R^8$ et $R^9$ représentent chacun un atome d'hydrogène et $R^{10}$ représente un radical cyano.

13. Dérivé du N-phénylpyrazole selon la revendication 11, caractérisée en ce qu'il est constitué par le 5-amino-4-cyano-1-(2,3,4-trichlorophényl)pyrazole.

14. Procédé pour la préparation d'un dérivé du N-phénylpyrazole selon la revendication 11, dans laquelle les symboles $R^5$, $R^6$, $R^7$, $R^8$ et $R^9$ sont comme définis à la revendication 1, et $R^{10}$ représente un radical cyano, à l'exclusion des 5 - amino - 4 - cyano - 1 - (2,4 - dichlorophényl)pyrazole, 5 - amino - 4 - cyano - 1 - (2,4,5 - trichlorophényl)pyrazole, 5 - amino - 4 - cyano - 1 - (4 - chloro - 2 - méthylphényl)-pyrazole, 5 - amino - 4 - cyano - 1 - (2,4 - dinitrophényl)pyrazole, et 5 - amino - 4 - cyano - 1 - (2,4,6 - trichlorophényl)pyrazole, caractérisé en ce qu'on effectue:

A) La cyclisation du composé de formule générale:

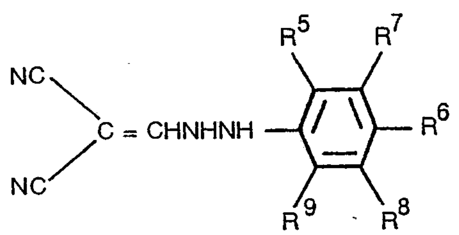

IV

dans laquelle les divers substituants sont définis comme dans la revendication 1, sous réserve que:

i) $R^5$ et $R^6$ sont autres que des atomes de chlore, lorsque $R^7$, $R^8$ et $R^9$ sont chacun un atome d'hydrogène,

ii) $R^5$ est autre que le radical méthyle, lorsque $R^6$ est un atome de chlore et $R^7$, $R^8$ et $R^9$ sont chacun un atome d'hydrogène,

iii) $R^5$ est autre qu'un atome de chlore lorsque $R^6$ et $R^8$ représentent chacun un atome de chlore et $R^7$ et $R^9$ représentent chacun un atome d'hydrogène,

iv) $R^5$ est autre qu'un radical nitro lorsque $R^6$ est un radical nitro et $R^7$, $R^8$ et $R^9$ représentent chacun un atome d'hydrogène, et

v) $R^5$ est autre qu'un atome de chlore quand $R^6$ et $R^9$ représentent chacun un atome de chlore et $R^7$ et $R^8$ représentent chacun un atome d'hydrogène, ou

B) La réaction d'un composé de formule générale:

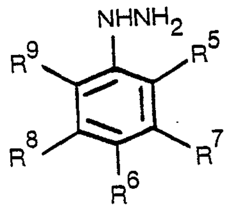

V

dans laquelle les divers substituants sont définis comme dans la revendication 1, sous réserve que:

i) $R^5$ et $R^6$ sont autres que des atomes de chlore, lorsque $R^7$, $R^8$ et $R^9$ sont chacun un atome d'hydrogène,

ii) $R^5$ est autre que le radical méthyle, lorsque $R^6$ est un atome de chlore et $R^7$, $R^8$ et $R^9$ sont chacun un atome d'hydrogène,

iii) $R^5$ est autre qu'un atome de chlore lorsque $R^6$ et $R^8$ représentent chacun un atome de chlore et $R^7$ et $R^9$ représentent chacun un atome d'hydrogène,

iv) $R^5$ est autre qu'un radical nitro lorsque $R^6$ est un radical nitro et $R^7$, $R^8$ et $R^9$ représentent chacun un atome d'hydrogène, et

v) $R^5$ est autre qu'un atome de chlore quand $R^6$ et $R^9$ représentent chacun un atome de chlore et $R^7$ et $R^8$ représentent chacun un atome d'hydrogène, ou un sel d'acide, avec un composé de formule générale:

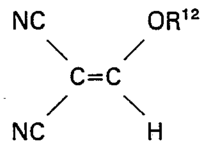

(VI)

dans laquelle $R^{12}$ représente un radical alcoyle linéaire ou ramifié contenant de 1 à 4 atomes de carbone, cette réaction étant éventuellement suivie d'une étape de conversion en un sel d'acide, par des méthodes en soi connues, du dérivé de N-phénylpyrazole ainsi obtenu ayant la formule générale décrite à la revendication 1, dans laquelle au moins un des symboles $R^5$, $R^6$, $R^7$, $R^8$ et $R^9$ représente un radical amino primaire.

15. Procédé pour la préparation d'un dérivé du N-phénylpyrazole selon la revendication 11, dans laquelle $R^5$, $R^6$, $R^7$, $R^8$, et $R^9$ sont définis comme à la revendication 1, sauf pour les radicaux amino primaires et cyano, et $R^{10}$ représente un groupe carbamoyle substitué

$$—CONHR^{11}$$

(dans lequel $R^{11}$ est comme défini à la revendication 1), caractérisé en ce qu'il comprend l'alcoylation sélective d'un composé de formule générale:

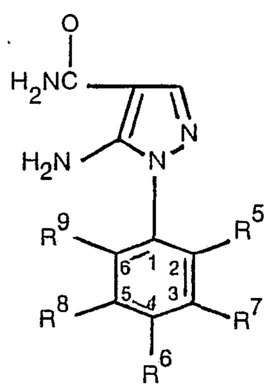

VII

(dans laquelle $R^5$, $R^6$, $R^7$, $R^8$, et $R^9$ sont comme définis à la revendication 1, sauf pour les radicaux amino primaires et cyano), avec l'iodure de méthyle ou d'éthyle, en présence d'une base minérale.

16. Procédé pour la préparation d'un dérivé du N-phénylpyrazole selon la revendication 11, dans laquelle $R^5$, $R^6$, $R^7$, $R^8$ et $R^9$ sont définis comme à la revendication 1 et $R^{10}$ représente un radical carbamoyle substitué

$$—CONHR^{11}$$

(dans lequel $R^{11}$ est défini comme à la revendication 1), caractérisé en ce qu'on fait réagir un composé de formule générale:

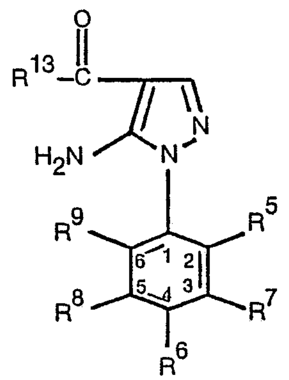

VIII

dans laquelle $R^5$, $R^6$, $R^7$, $R^8$, et $R^9$ sont comme définis à la revendication 1 et $R^{13}$ représente un atome de chlore ou un radical alcoxy linéaire ou ramifié contenant de 1 à 4 atomes de carbone, avec une amine de formule générale

$$H_2NR^{11} \qquad IX$$

dans laquelle $R^{11}$ représente un radical méthyle ou éthyle, cette réaction étant éventuellement suivie d'un étape de conversion en un sel d'acide, par des méthodes en soi connues, du dérivé de N-phénylpyrazole ainsi obtenu ayant la formule générale décrite à la revendication 1, dans laquelle au moins un des symboles $R^5$, $R^6$, $R^7$, $R^8$ et $R^9$ représente un radical amino primaire.

17. Composé de formule générale IV décrite à la revendication 14 dans laquelle les divers substituants sont définis comme dans la revendication 11, sous réserve que:

i) $R^5$ et $R^6$ sont autres que des atomes de chlore, lorsque $R^7$, $R^8$ et $R^9$ sont chacun un atome d'hydrogène,

ii) $R^5$ est autre que le radical méthyle, lorsque $R^6$ est un atome de chlore et $R^7$, $R^8$ et $R^9$ sont chacun un atome d'hydrogène,

iii) $R^5$ est autre qu'un atome de chlore lorsque $R^6$ et $R^8$ représentent chacun un atome de chlore et $R^7$ et $R^9$ représentent chacun un atome d'hydrogène,

iv) $R^5$ est autre qu'un radical nitro lorsque $R^6$ est un radical nitro et $R^7$, $R^8$ et $R^9$ représentent chacun un atome d'hydrogène, et

v) $R^5$ est autre qu'un atome de chlore quand $R^6$ et $R^9$ représentent chacun un atome de chlore et $R^7$ et $R^8$ représentent chacun un atome d'hydrogène.

**Revendications pour l'Etat contractant: AT**

1. Composition herbicide caractérisée en ce qu'elle contient comme matière active, au moins un dérivé du N-phénylpyrazole de formule générale III:

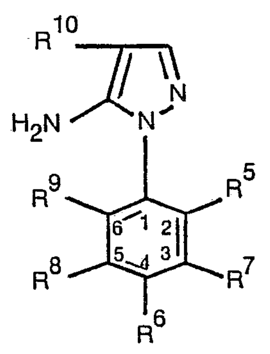

III

(dans laquelle:

— $R^5$ et $R^6$, identiques ou différents, représentent chacun un radical alcoyle ou alcoxy contenant de 1 à 4 atomes de carbone, un radical trifluorométhyle, trifluorométoxy, nitro, cyano ou amino primaire, ou un atome de fluor, chlore ou brome,

— $R^7$, $R^8$, et $R^9$, identiques ou différents, représentent chacun un atome d'hydrogène, un radical alcoyle ou alcoxy contenant de 1 à 4 atomes de carbone, un radical trifluorométhyle, trifluorométhoxy, nitro, cyano ou amino primaire, ou un atome de fluor, chlore ou brome,

— $R^5$, $R^7$, $R^8$ et $R^9$ pouvant de plus représenter chacun un atome d'hydrogène et $R^6$ le radical trifluorométhoxy ou trifluorométhyle,

— $R^{10}$ représente un radical cyano ou un radical carbamoyle substitué

$$—CONHR^{11}$$

dans lequel $R^{11}$ représente un radical méthyle ou éthyle), ou, lorsque au moins un des symboles $R^5$, $R^6$, $R^7$, $R^8$ et $R^9$ représente un radical amino primaire, un sel d'acide acceptable en agriculture, à l'exclusion du 5-amino-4-cyano-1-(2,4,6-trichlorophényl)pyrazole, en association avec un ou plus diluant ou support compatible et acceptable pour des herbicides, sous réserve que, quand le N-phénylepyrazole de formule générale III est le 5-amino-4-cyano-1-(2,4-dichlorophényl)pyrazole le 5-amino-4-cyano-1-(2,4,5-trichlorophényl)pyrazole, le 5-amino-4-cyano-1-(4-chloro-2-méthylphényl)pyrazole ou le 5-amino-4-cyano-1-(2,4-dinitrophényl)pyrazole, la composition herbicide n'est pas une association du dérivé N-phénylpyrazole seulement avec l'eau ou un solvant organique commun.

2. Composition herbicide selon la revendication 1 caractérisée en ce que la matière active est un dérivé du N-phénylpyrazole ayant la formule générale décrite à la revendication 1 dans laquelle:

— $R^5$ et $R^6$, identiques ou différents, représentent chacun un radical méthyle, trifluorométhyle, ou nitro ou un atome de fluor, chlore ou brome,

— $R^7$ représente un radical méthyle, méthoxy ou cyano ou un atome de fluor, chlore ou brome,

— $R^8$ et $R^9$ représentent chacun un atome d'hydrogène,

— $R^{10}$ représente un radical cyano.

3. Composition herbicide selon la revendication 1, caractérisée en ce que la matière active est le 5-amino-4-cyano-1-(2,3,4-trichlorophényl)pyrazole.

4. Composition herbicide selon l'une des revendications 1, 2 ou 3, caractérisée en ce qu'elle contient de 0,05 à 90% en poids du dérivé(s) du N-phénylpyrazole.

5. Composition herbicide selon la revendication 1, caractérisée en ce qu'elle se présente sous forme d'une suspension aqueuse concentrée contenant:

— de 10 à 70% en poids/volume d'au moins un dérivé du N-phénylpyrazole ayant la formule générale décrite à la revendication 1,

— de 2 à 10% en poids/volume d'un agent tensio-actif,

— de 0,1 à 5% en poids/volume d'un épaississant et

— de 15 à 87,9% en volume d'eau.

6. Composition herbicide selon la revendication 1, caractérisé en ce qu'elle se présente sous forme d'une poudre mouillable contenant:

— de 10 à 90% en poids d'au moins un dérivé du N-phénylpyrazole ayant la formule générale décrite à la revendication 1,

— de 2 à 10% en poids d'agent tensio-actif, et

— de 10 à 88% en poids d'un diluant ou un support solide.

7. Composition herbicide selon la revendication 1, caractérisée en ce qu'elle se présente sous forme d'un concentré liquide soluble dans l'eau contenant:

— de 10 à 30% en poids/volume d'au moins un dérivé du N-phénylpyrazole ayant la formule générale décrite à la revendication 1,

— de 5 à 25% en poids/volume d'agent tensio-actif, et

— de 45 à 85% en volume de solvant miscible à l'eau.

8. Composition herbicide selon la revendication 1, caractérisée en ce qu'elle se présente sous forme d'une suspension concentrée liquide émulsionnable contenant:

— de 10 à 70% en poids/volume d'au moins un dérivé du N-phénylpyrazole ayant la formule générale décrite à la revendication 1,

— de 5 à 15% en poids/volume d'agent tensio-actif,

— de 0,1 à 5% en poids/volume d'épaississant et

— de 10 à 84,9% en volume de solvant organique.

9. Composition herbicide selon la revendication 1, caractérisée en ce qu'elle se présente sous forme de granulés contenant:

— de 2 à 10% en poids/volume d'au moins un dérivé du N-phénylpyrazole ayant la formule générale décrite à la revendication 1,

— de 0,5 à 2% en poids d'agent tensio-actif,

— et de 88 à 97,5% en poids de support granulé.

10. Procédé de contrôle de la croissance des adventices en un lieu donné, caractérisé en ce qu'on applique en ce lieu, une composition herbicide selon l'une des revendications 1, 2, 3 et 4, à raison de 0,1 à 20 kg/ha de dérivé N-phénylpyrazole.

11. Procédé pour la préparation d'un dérivé du N-phénylpyrazole ayant la formule générale décrite à la revendication 1, dans laquelle les symboles $R^5$, $R^6$, $R^7$, $R^8$ et $R^9$ sont comme définis à la revendication 1, et $R^{10}$ représente un radical cyano, à l'exclusion des 5 - amino - 4 - cyano - 1 - (2,4 - dichlorophényl)pyrazole, 5 - amino - 4 - cyano - 1 - (2,4,5 - trichlorophényl)pyrazole, 5 - amino - 4 - cyano - 1 - (4 - chloro - 2 - méthylphényl)pyrazole, 5 - amino - 4 - cyano - 1 - (2,4 - dinitrophényl)pyrazole, et 5 -

amino - 4 - cyano - 1 - (2,4,6 - trichlorophényl)pyrazole, caractérisé en ce qu'on effectue:

A) La cyclisation du composé de formule générale:

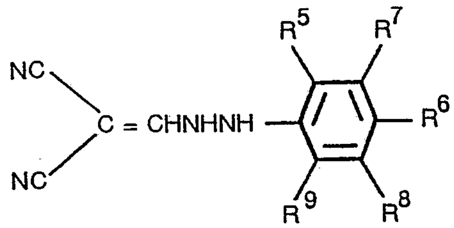

IV

dans laquelle les divers substituants sont définis comme dans la revendication 1, sous réserve que:

i) $R^5$ et $R^6$ sont autres que des atomes de chlore, lorsque $R^7$, $R^8$ et $R^9$ sont chacun un atome d'hydrogène,

ii) $R^5$ est autre que le radical méthyle, lorsque $R^6$ est un atome de chlore et $R^7$, $R^8$ et $R^9$ sont chacun un atome d'hydrogène,

iii) $R^5$ est autre qu'un atome de chlore lorsque $R^6$ et $R^8$ représentent chacun un atome de chlore et $R^7$ et $R^9$ représentent chacun un atome d'hydrogène,

iv) $R^5$ est autre qu'un radical nitro lorsque $R^6$ est un radical nitro et $R^7$, $R^8$ et $R^9$ représentent chacun un atome d'hydrogène, et

v) $R^5$ est autre qu'un atome de chlore quand $R^5$ et $R^9$ représentent chacun un atome de chlore et $R^7$ et $R^8$ représentent chacun un atome d'hydrogène, ou

B) La réaction d'un composé de formule générale:

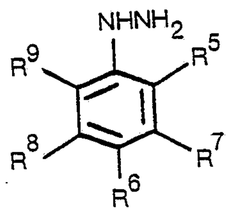

V

dans laquelle les divers substituants sont définis comme dans la revendication 1, sous réserve que:

i) $R^5$ et $R^6$ sont autres que des atomes de chlore, lorsque $R^7$, $R^8$ et $R^9$ sont chacun un atome d'hydrogène,

ii) $R^5$ est autre que le radical méthyle, lorsque $R^6$ est un atome de chlore et $R^7$, $R^8$ et $R^9$ sont chacun un atome d'hydrogène,

iii) $R^5$ est autre qu'un atome de chlore lorsque $R^6$ et $R^8$ représentent chacun un atome de chlore et $R^7$ et $R^9$ représentent chacun un atome d'hydrogène,

iv) $R^5$ est autre qu'un radical nitro lorsque $R^6$ est un radical nitro et $R^7$, $R^8$ et $R^9$ représentent chacun un atome d'hydrogène, et

v) $R^5$ est autre qu'un atome de chlore quand $R^6$ et $R^9$ représentent chacun un atome de chlore et $R^7$ et $R^8$ représentent chacun un atome d'hydrogène, ou un sel d'acide, avec un composé de formule générale:

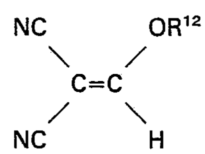

(VI)

dans laquelle $R^{12}$ représente un radical alcoyle linéaire ou ramifié contenant de 1 à 4 atomes de carbone, cette réaction étant éventuellement suivie d'une étape de conversion en un sel d'acide, par des méthodes en soi connues, du dérivé de N-phénylpyrazole ainsi obtenu ayant la formule générale décrite à la revendication 1, dans laquelle au moins un des symboles $R^5$, $R^6$, $R^7$, $R^8$ et $R^9$ représente un radical amino primaire.

12. Procédé pour la préparation d'un dérivé du N-phénylpyrazole ayant la formule générale décrite a la revendication 1, dans laquelle $R^5$, $R^6$, $R^7$, $R^8$, et $R^9$ sont définis comme à la revendication 1, sauf pour les radicaux amino primaires et cyano, et $R^{10}$1représente un groupe carbamoyle substitué

$$—CONHR^{11}$$

(dans lequel $R^{11}$ est comme défini à la revendication 1), caractérisé en ce qu'il comprend l'alcoylation sélective d'un composé de formule générale:

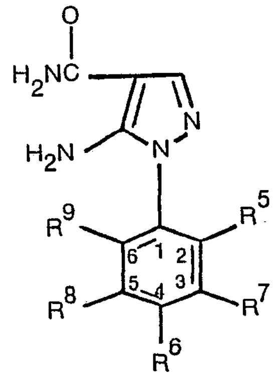

VII

(dans laquelle $R^5$, $R^6$, $R^7$, $R^8$, et $R^9$ sont comme définis à la revendication 1, sauf pour les radicaux amino primaires et cyano), avec l'iodure de méthyle ou d'éthyle, en présence d'une base minérale.

13. Procédé pour la préparation d'un dérivé du N-phénylpyrazole ayant la formule générale décrite à la revendication 1, dans laquelle $R^5$, $R^6$, $R^7$, $R^8$ et $R^9$ sont définis comme à la revendication 1 et $R^{10}$ représente un radical carbamoyle substitué

$$—CONHR^{11}$$

(dans lequel $R^{11}$ est défini comme à la revendication 1), caractérisé en ce qu'on fait réagir un composé de formule générale:

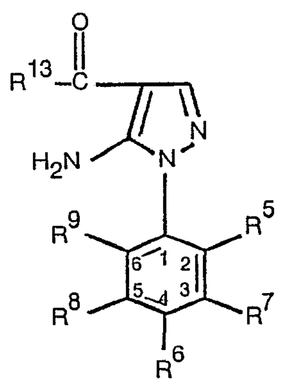

VIII

dans laquelle $R^5$, $R^6$, $R^7$, $R^8$, et $R^9$ sont comme définis à la revendication 1 et $R^{13}$ représente un atome de chlore ou un radical alcoxy linéaire ou ramifié contenant de 1 à 4 atomes de carbone, avec une amine de formule générale

$$H_2NR^{11} \qquad IX$$

dans laquelle $R^{11}$ représente un radical méthyle ou éthyle, cette réaction étant éventuellement suivie d'une étape de conversion en un sel d'acide, par des méthodes en soi connues, du dérivé de N-phénylpyrazole ainsi obtenu ayant la formule générale décrite à la revendication 1, dans laquelle au moins un des symboles $R^5$, $R^6$, $R^7$, $R^8$ et $R^9$ représente un radical amino primaire.